# EUROPEAN PATENT SPECIFICATION

(11) **EP 4 566 616 B1**
(45) Date of publication and mention of the grant of the patent: **08.07.2026**
(21) Application number: 24216443.2
(22) Date of filing: 29.11.2024
(51) Int. Cl.: A61K 38/00, C08F 8/32, C08F 8/34, C08F 257/02, G01N 33/00

(54) **PARTICLE FOR USE IN AGGLUTINATION METHOD, TEST PARTICLE, REAGENT, TEST KIT, METHOD FOR PRODUCING PARTICLE, AND METHOD FOR DETECTING TARGET SUBSTANCE**
PARTIKEL ZUR VERWENDUNG IN EINEM AGGLUTINIERUNGSVERFAHREN, TESTPARTIKEL, REAGENS, TESTKIT, VERFAHREN ZUR HERSTELLUNG DES PARTIKELS UND VERFAHREN ZUM NACHWEIS EINER ZIELSUBSTANZ
PARTICULE DESTINÉE À ÊTRE UTILISÉE DANS UN PROCÉDÉ D'AGGLUTINATION, PARTICULE DE TEST, RÉACTIF, KIT DE TEST, PROCÉDÉ DE PRODUCTION DE PARTICULE ET PROCÉDÉ DE DÉTECTION DE SUBSTANCE CIBLE

(30) Priority: 30.11.2023 JP 2023203389; 19.11.2024 JP 2024201623
(43) Date of publication of application: 11.06.2025
(73) Proprietor: CANON KABUSHIKI KAISHA, Tokyo 146-8501 (JP)
(72) Inventor: SUDA, Sakae, Tokyo (JP); TSUNODA, Masaki, Tokyo (JP); ASANO, Ibuki, Tokyo (JP); NATORI, Ryo, Tokyo (JP); KAWAMURA, Masashi, Tokyo (JP); UMEDA, Noriyoshi, Tokyo (JP)
(74) Representative: WESER & Kollegen Patentanwälte PartmbB

(56) References cited:
- CN-A- 114 531 879
- JP-A- 2021 032 670
- US-B2- 11 597 787

## Description

### BACKGROUND OF THE INVENTION

### Field of the Invention

The present disclosure relates to a particle for use in an agglutination method, a test particle, a reagent, a test kit, a method for detecting a target substance, and a method for producing the particle.

### Description of the Related Art

In recent years, the agglutination method such as the immunolatex agglutination measurement method has attracted attention as a simple and rapid immunoassay method. A known method includes mixing a dispersion of particles having an antibody or antigen on the surface as a ligand with a sample that may contain a target substance (antigen or antibody). At this time, when the target substance (antibody or antigen) is contained in the sample, the particles will provoke an agglutination reaction, thus the presence or absence of disease can be identified by optically detecting this agglutination reaction as a change in the amount of scattered light intensity, transmitted light intensity, absorbance and the like. It is desirable that the property of adsorbing a substance other than the target substance, a so-called non-specific adsorption property, of the particle used in the agglutination method is small to reduce noise. As particles used in the agglutination method, polystyrene particles are widely used, and particles having a structure derived from styrene and glycidyl methacrylate are also known. Patent Literature 1 describes a particle having a core and shell structure, wherein the shell has a carboxy group that is a reactive functional group, and a 2,3-dihydroxypropyl group. It has been reported that, for this particle, the method of copolymerizing a monomer having a carboxy group and a monomer having a 2,3-dihydroxypropyl group into a seed particle (mother particle, core) of polystyrene, a styrene-based copolymer or the like by a two-stage swelling polymerization method to add a shell to the seed core to form a core and shell structure, and the method of using a monomer that can form a carboxy group or a 2,3-dihydroxypropyl group by hydrolysis to form a core and shell structure by a two-stage swelling polymerization method are preferred. CN 114 531 879 A, US 11 597 787 B2 and JP 2021 032670 A disclose particles comprising styrene, divinylbenzene, and glycidyl methacrylate, for which a content proportion of styrene is below 40% by mass and for which no shell thickness is disclosed.

### SUMMARY OF THE INVENTION

The present inventors have synthesized a particle having a core and shell structure according to Patent Literature 1 (Japanese Patent Application Laid-Open No. 2007-224213), prepared a dispersion of a particle in which an antibody or an antigen is chemically bound on the surface of the particle as a ligand, mixed the dispersion with a sample containing a target substance, and have checked the change in absorbance by an agglutination method. However, depending on the type and concentration of the target substance, the change in absorbance has been not sufficiently obtained, and the sensitivity has been low. Even when a particle having a core and shell structure has been synthesized with reduced amounts of glycidyl methacrylate to improve sensitivity, the desired sensitivity has not been obtained and non-specific adsorption has worsened.

In addition, when a copolymer particle of styrene and glycidyl methacrylate without having a core and shell structure has been made and used, the desired sensitivity has not been obtained either.

The present disclosure has been made in light of the foregoing background and problems. Specifically, an object of the present disclosure is to provide a particle capable of having both improved sensitivity and suppressed non-specific adsorption in an agglutination method and a production method thereof.

The present disclosure provides:
a particle for use in an agglutination method, the particle including a core particle and a shell on a surface of the core particle, wherein the core particle has a volume average particle size of 200 nm or more and 500 nm or less, as measurable by dynamic light scattering, and contains a polymer having a structural unit represented by formula (1), wherein a content proportion of the structural unit represented by the formula (1) to the particle is 40% by mass or more and 85% by mass or less; and the shell has a thickness of 5 nm or more and 50 nm or less, and contains a polymer having a structural unit represented by formula (2) 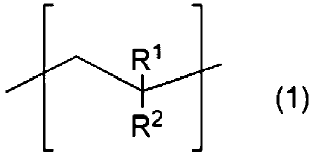
(wherein, R¹ represents a hydrogen atom or a methyl group; R² represents a substituted or unsubstituted phenyl or naphthyl group, provided that, when substituted, a substituent is a methyl group or an ethyl group; and R¹ and R² may be different for each structural unit) 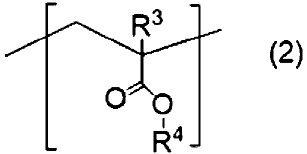
(wherein, R³ represents a hydrogen atom or a methyl group; R⁴ represents a group having an epoxy group, a group having a hydroxy group, or a group having a carboxy group; and R³ and R⁴ may be different for each structural unit).

Further features of the present invention will become apparent from the following description of exemplary embodiments.

### DESCRIPTION OF THE EMBODIMENTS

Preferred embodiments of the present invention will now be described in detail.

Hereinafter, the present disclosure will be described in detail below with reference to embodiments.

### First Embodiment

The present disclosure, as a first embodiment, provides the following particle.

A particle for use in an agglutination method, the particle including a core particle and a shell on a surface of the core particle, wherein
the core particle has a volume average particle size of 200 nm or more and 500 nm or less, and contains a polymer having a structural unit represented by formula (1), wherein a content proportion of the structural unit represented by the formula (1) to the particle is 40% by mass or more and 85% by mass or less; and
the shell has a thickness of 5 nm or more and 50 nm or less, and contains a polymer having a structural unit represented by formula (2) 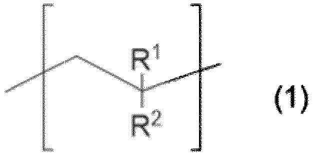
   (wherein, R¹ represents a hydrogen atom or a methyl group; R² represents a substituted or unsubstituted phenyl or naphthyl group, provided that, when substituted, a substituent is a methyl group or an ethyl group; and R¹ and R² may be different for each structural unit) 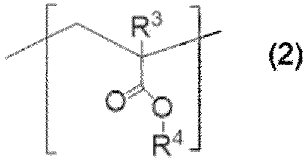
   (wherein, R³ represents a hydrogen atom or a methyl group; R⁴ represents a group having an epoxy group, a group having a hydroxy group, or a group having a carboxy group; and R³ and R⁴ may be different for each structural unit).

The particle according to the present embodiment can be said as a particle including a first layer and a second layer in this order. The first layer may be a particle, and it can be said that the aforementioned core particle is a first layer, and the aforementioned shell is a second layer. It should be noted that, in the present embodiment, a layer different from the second layer may be provided between the first layer and the second layer or further outside the second layer (on the surface side of the particle). Hereinafter, the particle according to the present embodiment will be described with an example of the configuration of the particle including a first layer and a second layer in this order, wherein the first layer is a core particle and the second layer is a shell.

The particle according to the present embodiment contains a polymer having a structure represented by formula (1), wherein a content of the structure represented by formula (1) is 40% by mass or more and 85% by mass or less of the core particle. The formula (1) has a substituted or unsubstituted phenyl or naphthyl group in R². The phenyl group and the naphthyl group are known to increase the refractive index. The refractive index of the particle is increased by having 40% by mass or more and 85% by mass or less of the repeating unit structure represented by formula (1) in the particle, and thus the refractive index of the particle itself is also increased. When a reagent containing a particle reacts with a target substance and agglutinates, the absorbance increases. When a particle with a higher refractive index is agglutinated, the absorbance becomes greater, thus the difference in absorbance before and after agglutination is also larger and the sensitivity is improved. It is preferable that the repeating unit structure represented by formula (1) is included in the particle by 50% by mass or more, and more preferably 60% by mass or more. It is also preferable the repeating unit structure represented by formula (1) is included in the particle by 65% by mass or less. In addition, it is preferable that a content proportion of the structural unit represented by the formula (1) to the core particle is 90% by mass or more.

It should be noted that the core particle may contain a polymerization initiator, a copolymer derived from a vinyl monomer, a color material such as a pigment, a dye or a fluorescent agent, an inorganic filler and the like. The content thereof is preferably 9% by mass or less.

The core particle of the present embodiment has a volume average particle size of 200 nm or more and 500 nm or less. In tests by the agglutination method, it is common to use light of a wavelength in the visible light region. In this wavelength region, as the particle size increases, the absorbance also increases until the particle size of 1 µm (Non Patent Literature 1; Medical Electronics and Bioengineering, Vol. 22, No. 4, 39-45 (1984)). Thus, when the particle of the present embodiment having a core particle size of 200 nm or more in volume average particle size and a shell having a thickness of 5 nm or more is agglutinated, the agglutinate of the particle after agglutination becomes larger and the absorbance between before and after the agglutination increases larger compared to when a particle having a core particle size of less than 200 nm is agglutinated, thus the sensitivity becomes high. Furthermore, when the core particle size is 500 nm or less, even if the concentration of a target substance is high, the sensitivity increases at a certain level or higher with the increase of the concentration, and thus the concentration of the target substance in the sample can be evaluated. Note that the particle according to the present embodiment is used in an agglutination method. The agglutination method can be referred to as a turbidimetric method in the sense of detecting a change in turbidity before and after mixing a target substance with a particle. The present embodiment can also be referred to as a particle for use in an immunoturbidimetric method when the particle is bound to an antibody or an antigen to detect an antigen or an antibody.

In the particle of the present embodiment, the shell has a thickness of 5 nm or more and 50 nm or less, and contains a polymer having a structural unit represented by formula (2). The structure represented by formula (2) is highly hydrophilic because it has any of an epoxy group, a hydroxy group or a carboxy group. By covering the surface of the core particle with a shell layer containing a polymer having the structure represented by formula (2), the hydrophobic portion of the surface of the particles is covered, and the hydrophilicity is increased. As a result, hydrophobic interactions with proteins other than the target substance in the sample are reduced, and non-specific adsorption can be suppressed.

In addition, when the thickness of the shell is 5 nm or more and 50 nm or less, the particle can be covered without exposing the hydrophobic surface of the core particle, the dispersity of the particles is not too high and the agglutination of the particles is not easily suppressed, so that the sensitivity becomes high, and thus both high sensitivity and suppression of non-specific adsorption can be achieved.

In this embodiment, a content proportion of the structural unit represented by the formula (2) to the core particle is preferably 9% by mass or more and 60% by mass or less, more preferably 9% by mass or more and 53% by mass or less, more preferably 9% by mass or more and 39% by mass or less, more preferably 16% by mass or more and 39% by mass or less, more preferably 20% by mass or more and 39% by mass or less, more preferably 31% by mass or more and 39% by mass or less.

In the present embodiment, the content of the structure represented by formula (2) is 9% by mass or more and 60% by mass or less in the particle, so that the hydrophobicity of the particle surface is reduced and non-specific adsorption can be suppressed.

Furthermore, the dispersity of the particles is not too high and the agglutination of the particles is not easily suppressed, so that the sensitivity becomes high, and thus both high sensitivity and suppression of non-specific adsorption can be achieved.

The particle of the present embodiment has a structure including a core particle and a shell covering the core particle, wherein the core has a high refractive index and a large absorbance difference before and after agglutination and the shell is highly hydrophilic and can suppress non-specific adsorption, as described below. By having such a core and shell structure, the particle of the present embodiment can have both high refractive index and hydrophilicity of the particle surface and enables balancing the high-sensitivity detection and suppression of non-specific adsorption in the agglutination method.

In the present embodiment, it is preferable that the formula (1) is a structure represented by formula (1-A). With formula (1-A), the core refractive index of the core particle can be improved. In addition, since the structure of formula (1-A) is highly hydrophobic, the hydrophilic shell component is less likely to be incorporated into the core part 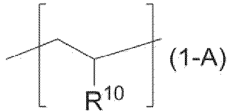 (wherein, R¹⁰ represents a phenyl group, a tolyl group or a naphthyl group).

The structure represented by formula (1-A) in the present embodiment is obtained by polymerizing the monomer represented by formula (X1) described below. Specific examples of the monomer include styrenes, 1-vinylnaphthalene and 2-vinylnaphthalene as described below, and in particular, styrene, 1-vinylnaphthalene and 2-vinylnaphthalene are preferred. These monomers may be used singly, or a plurality of these monomers may be used at the same time.

It is preferable that the core particle further has a crosslinked structure. The crosslinked structure is obtained by polymerizing a crosslinkable, radically polymerizable monomer, which is a monomer having two or more radically polymerizable unsaturated bonds in one molecule. Examples of such a crosslinkable monomer include multifunctional (meth)acrylates such as ethylene glycol diacrylate, ethylene glycol dimethacrylate, trimethylolpropane triacrylate, trimethylolpropane trimethacrylate, pentaerythritol triacrylate, pentaerythritol trimethacrylate, dipentaerythritol hexaacrylate and dipentaerythritol hexamethacrylate; conjugated diolefins such as butadiene and isoprene; divinylbenzene; diallyl phthalate; allyl acrylate, and allyl methacrylate. Two or more types of crosslinkable, radically polymerizable monomers may be used. The structure represented by formula (3) is more preferable as a crosslinked structure. When the core particle has the crosslinked structure, the particle having the core and shell structure become physically stronger, and there is no concern of cracking or chipping even when the centrifugal operation is repeated during purification. 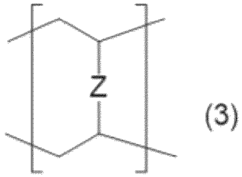 (wherein, Z represents a substituted or unsubstituted phenylene or naphthalene group, provided that, when substituted, a substituent is a methyl group or an ethyl group. Z may be different for each structural unit.)

Examples of the crosslinkable, radically polymerizable monomer used to form the crosslinked structure of formula (3) include 1,2-divinylbenzene, 1,3-divinylbenzene, 1,4-divinylbenzene, 2,6-diethynylnaphthalene and 2,7-diethynylnaphthalene. These may be used singly, or a plurality of them may be used at the same time.

Among the mentioned crosslinkable, radically polymerizable monomers as examples, divinylbenzene is preferred.

Although the reason is not clear, when divinylbenzene is used, handling during the radical polymerization reaction is excellent, the monomer conversion rate during core particle formation is improved, and the core component is less likely to be incorporated into the shell, and thus it is preferable.

In the present embodiment, it is preferable that the mass ratio of the core to the particle is 50% by mass or more and 95% by mass or less. When the mass ratio of the core to the particle is 50% by mass or more, the content of the core particle having high refractive index can be increased, the refractive index can be improved, the absorbance difference before and after agglutination can be increased, and the sensitivity in the region where the concentration of the target substance is low can be improved. When the mass ratio of the core to the particle is 95% by mass or less, the surface of the core particle can be covered with the shell layer, the hydrophilicity of the surface of the particle can be increased, and suppression of non-specific adsorption can be improved.

In the present embodiment, it is preferable that the formula (2) is a structure represented by formula (2-A). The structure represented by formula (2-A) has one of a hydroxy group and a carboxy group, and the ability to suppress non-specific adsorption is equivalent to or greater than the structure having an epoxy group, which is preferable: 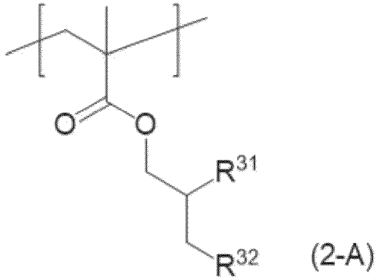
(wherein, one of R³¹ and R³² represents a hydroxy group, and the other represents a hydroxy group, a group represented by formula (2-B) or a group represented by formula (2-C)) 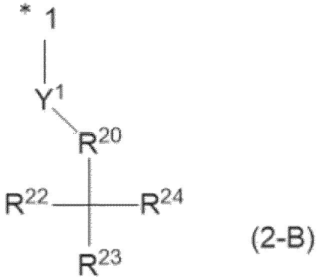
(wherein, R²⁰ represents a single bond or a methylene group; R²², R²³ and R²⁴ each represent a hydrogen atom, a methyl group, a hydroxy group or a hydroxymethyl group, and one or more of R²², R²³ and R²⁴ represent a hydroxy group; Y¹ represents a sulfur atom or an imino group; and *1 represents a bonding position), 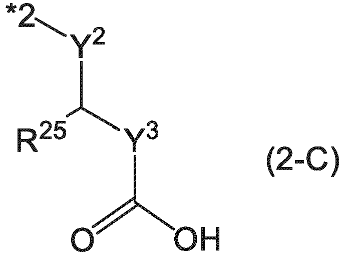
(wherein, R²⁵ represents a hydrogen atom, a methyl group, a hydroxy group or a carboxy group; Y² represents a sulfur atom or an imino group; Y³ represents a single bond or a methylene group; and *2 represents a bonding position).

When the total of the number of the hydroxy groups (0 or more) and carboxy groups (0 or more) in the formula (2-A) is 2 or more, non-specific adsorption can be decreased even under the condition where the thickness of the shell is 5 nm or more and 50 nm or less because a structure with high hydrophilicity is included in one structural unit. Furthermore, it is more preferable that the total of the number of the hydroxy groups (0 or more) and carboxy groups (0 or more) in the formula (2-A) is 3 or more in terms of the even higher hydrophilicity.

Specific examples of the structure of formula (2-A) include, but are not limited to, the following formulas (2-A-1) to (2-A-12). 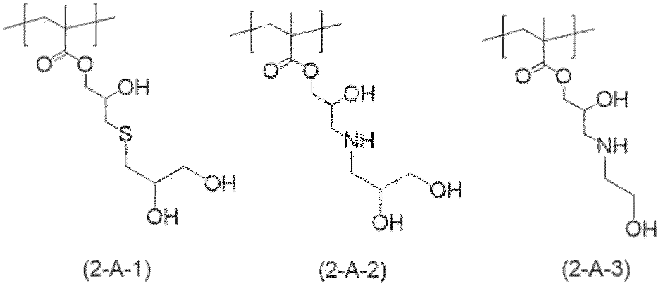 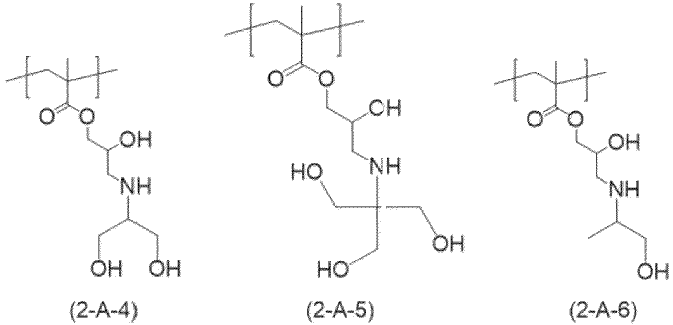 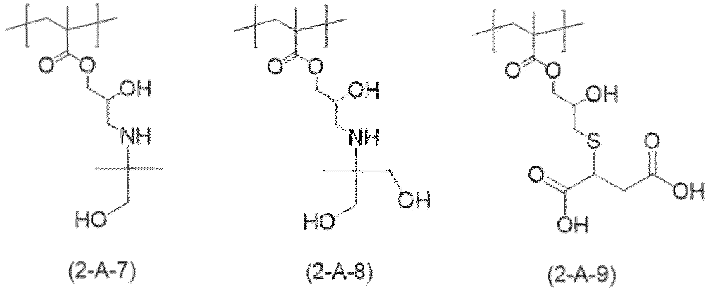 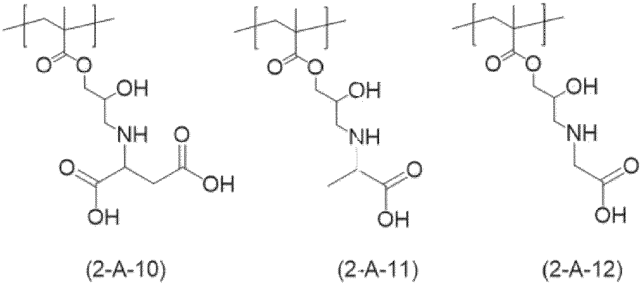 The structure represented by formula (2-A) in the present embodiment is obtained by reacting a polymer obtained by polymerizing a monomer represented by formula (X2) described below with formula (X3) described below.

As represented by formulae (2-A-1) to (2-A-12), the side chain may contain a sulfur atom or a nitrogen atom. Containing a sulfur atom in the side chain is more preferable from the viewpoint of sensitivity improvement. Containing a nitrogen atom in the side chain is more preferable from the viewpoint of suppression of non-specific adsorption. Since the structure indicated by "-S-" has a weak hydrophobic tendency, it is expected to moderately weaken the water-binding force of the highly hydrophilic side chain and suppress osmotic aggregation that may occur when the structure indicated by "-S-" is mixed with a high-concentration sample, thereby contributing to the improvement of sensitivity. Since the amino group has a hydrophilic tendency, it can contribute to the suppression of non-specific adsorption.

Specific examples of the monomer represented by (X2) are not particularly limited, but glycidyl (meth)acrylate is preferable.

It is preferable that the content proportion of the structural unit represented by formula (2) to the shell is 90% by mass or more and 100% by mass or less. By setting the content of the structure represented by formula (2) to 90% by mass or more, the hydrophilicity of the shell can be increased and suppression of non-specific adsorption can be improved. To further suppress non-specific adsorption, it is more preferable that the content of the structure represented by formula (2) is 95% by mass or more. It should be noted that the shell may contain a polymerization initiator, a copolymer derived from a vinyl monomer, a color material such as a pigment, a dye or a fluorescent agent, an inorganic filler and the like. The content thereof is preferably 9% by mass or less.

The particle of the present embodiment may have a structure other than formula (1) or formula (2). Examples of the structure other than formula (1) and formula (2) included in the particle include, but are not particularly limited to, structures obtained by polymerizing monomers such as styrenes, acrylics and methacrylics. The particle of the present embodiment may have a plurality of structures other than formula (1) and formula (2) at the same time.

It is preferable that the composition ratio of C element to O element of the particle quantified when measured by X-ray photoelectron spectroscopy (XPS) is in the range of 2.0 or more and 3.3 or less. In XPS measurement, photoelectrons generated at from the outermost surface to about 10 nm are detected, thus XPS is a method that can analyze the components of the surface layer of the particle. When the composition ratio is less than 2.0, O elements in the surface layer of the particle are overabundance, and while hydroxyl and carboxyl groups are sufficiently attached, the hydrophilicity is very high, so that the particle is difficult to be agglutinated, and the detection sensitivity according to the principle of the agglutination method may be reduced. When the composition ratio is more than 3.3, O elements of the surface layer of the particle is too few, and the hydrophilicity is lacking, which not only is disadvantageous for the dispersion stability of the particle when stored for a long time, but also may worsen non-specific adsorption. Thus, by optimizing the composition ratio of the components of the surface layer of the particle, a particle having both excellent sensitivity and excellent non-specific adsorption can be provided.

By measuring the test particles of the present embodiment by FT-IR, the ratio of the formula (1) to the formula (2) can be evaluated. It is preferable that the measurement method is ATR method in which infrared light can be infiltrated from the outermost surface to about µm size to obtain FT-IR spectrum. In the infrared absorption spectrum (FT-IR spectrum), when the peak height present at 1500 to 1650 cm⁻¹ derived from the C=C bond of the aromatic ring is defined as A and the peak height present at 1680 to 1750 cm⁻¹ derived from the carbonyl group of the ester bond is defined as B, it is preferable that the particle of the present embodiment has a value of A/B of 0.85 or more and 5.35 or less in terms of balancing the sensitivity and the suppression of non-specific adsorption.

When the value of A/B is 0.85 or more, the proportion of formula (1) to the carbonyl group is sufficiently high, so that the detection sensitivity is improved. When the value of A/B is 5.35 or less, the carbonyl group of the ester bond contained in the particle is sufficiently abundant, that is, the proportion of the hydrophilic formula (2) to the whole particle is large. Thus, the particle gains hydrophilicity, and the non-specific adsorption property is improved.

The particle size of the particle of the present embodiment is 210 nm or more and 600 nm or less, more preferably 230 nm or more and 500 nm or less, in volume average particle size in an aqueous dispersion. When the particle is of 230 nm or more, the agglutinate of the particle after agglutination becomes larger compared to a particle of less than 230 nm, and the difference in absorbance before and after the agglutination increases and thus the sensitivity becomes high. When the particle size is 600 nm or less, even if the concentration of a target substance is high, the sensitivity increases at a certain level or higher with the increase of the concentration, and thus the concentration of the target substance in the sample can be evaluated.

The value of the ratio (Dv/Dn) of the volume average particle size (Dv) to the number average particle size (Dn) of the particle of the present embodiment is preferably 1.25 or less. It is known that the particle size distribution becomes narrower as the value of Dv/Dn approaches 1. When the value of Dv/Dn is 1.25 or less, the size of the particles varies little, and particles with large particle sizes are not included, so that the difference in size to the agglutinate after agglutination becomes large, and the sensitivity becomes high.

When the particle is dispersed in water so that the value of Dv/Dn is 1.25 or less at a solid content concentration of 0.1% by mass, and an absorbance at a cell length of 10 mm in an incident light of a wavelength of 572 nm is defined as E, it is preferable that the value of E/Dv is 8.0 × 10⁻⁴ or more and 2.0 × 10⁻³ or less. When the value of E/Dv is 8.0 × 10⁻⁴ or more and 2.0 × 10⁻³ or less, even if the particle size is a certain size or larger, the absorbance is large, and the difference in absorbance before and after agglutination becomes large, so that the sensitivity becomes high.

As a method for producing the core particle, a soap-free emulsion polymerization method is preferred. When a soap-free emulsion polymerization method is employed, the particle size distribution becomes uniform, the sensitivity is stable, and the detection limit can be improved in a region where the concentration of the target substance is low.

The method for forming the core and shell structure is not particularly limited as long as the core and shell structure is formed, but a method of adding a monomer that forms a shell to a dispersion of core particles, then adding a water-soluble polymerization initiator to form a shell structure is preferable in terms of the improvement of sensitivity and suppression of non-specific adsorption. Although the details are not clear, the mechanism thereof can be believed as follows. When the monomer and the water-soluble polymerization initiator are added to the aqueous dispersion of the core particles, the monomer dissolved in water begins to polymerize and an oligomer is formed. As the polymerization progresses, the hydrophobicity of the oligomer increases, and the oligomer becomes hard to be dispersed alone and is deposited on the surface of the core particle. On the surface of the particle, the monomer and the oligomer incorporated on the core particle surface further react to form a core and shell structure. In the particle thus formed, the shell component is less likely to penetrate into the core region, so that the reduction of the refractive index of the core particle therefor is less likely to occur. As a result, the absorbance difference before and after agglutination becomes large, and the sensitivity is improved. On the other hand, a two-stage swelling polymerization method in which a monomer that forms a shell on a core particle is swelled and a shell structure is formed with a hydrophobic initiator or the like is known as an effective method to uniform a particle size distribution, but in this method, the core particle swells. Thus, the shell component may penetrate into the core particle, which may cause the reduction of the refractive index of the core particle. Thus, when the particles produced by this method are used in an agglutination method, the sensitivity is difficult to be improved.

### Second Embodiment

The present disclosure provides, as a second embodiment, the following method for producing a particle for use in an agglutination method.

A method for producing a particle for use in an agglutination method, the method including:
a first step of performing a polymerization reaction of a first monomer composition containing a monomer represented by formula (X1) to obtain a core particle having a volume average particle size of 200 nm or more and 500 nm or less;
a second step of performing a polymerization reaction using a reaction solution comprising the core particle, a second monomer composition comprising a monomer represented by formula (X2) and a water-soluble polymerization initiator to obtain a particle having a layer of 5 nm or more and 50 nm or less formed outside of the core particle,
a content portion of the monomer represented by the formula (X1) to a total amount of the monomer contained in the first monomer composition is 90 mass% or more
provided that,
a content proportion of an unpolymerized monomer represented by the formula (X1) to the reaction solution is 1000 ppm or less, and
a content proportion of the monomer represented by the formula (X2) to a total amount of a monomer contained in the first monomer composition and a monomer contained in the second monomer composition is 9% by mass or more and less than 40% by mass; and
a third step of reacting the particle having the layer with a compound represented by formula (X3) 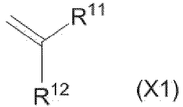
   (wherein, R¹¹ represents a hydrogen atom or a methyl group; and R¹² represents a substituted or unsubstituted phenyl or naphthyl group, provided that, when substituted, a substituent is a methyl group or an ethyl group) 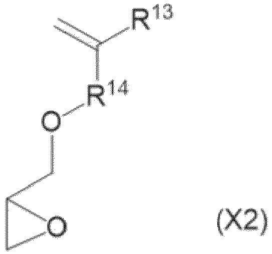
   (wherein, R¹³ represents hydrogen or a methyl group, and R¹⁴ represents an ethylene group or a carbonyl group) 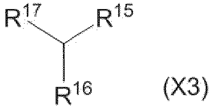
   (wherein, R¹⁵ represents an amino group or a thiol group; R¹⁶ and R¹⁷ each represent a hydrogen atom, a methyl group, a group having a hydroxy group or a group having a carboxy group; and at least one of R¹⁶ and R¹⁷ represents a group having a hydroxy group or a group having a carboxy group).

It is preferable that the content of the monomer represented by formula (X1) in the reaction solution before the start of the second step is 1000 ppm or less. When the content is 1000 ppm or less, the amount of the hydrophobic monomer represented by formula (X1) is small during forming the shell in the second step, and thus the hydrophobic repeated structure is less likely incorporated into the shell, and the non-specific adsorption property can be suppressed.

The water-soluble polymerization initiator used in the present embodiment is not particularly limited, but a water-soluble azo compound or a water-soluble peroxide is preferably used.

Preferred examples of the water-soluble azo compound include any of 4,4'-azobis(4-cyanovaleric acid), 2,2'-azobis[2-methyl-N-(2-hydroxyethyl)propionamide], 2,2'-azobis[N-(2-carboxyethyl)-2-methylpropionamidine]tetrahydrate, 2,2'-azobis(2-methylpropionamidine)dihydrochloride, 2,2'-azobis[2-(2-imidazolin-2-yl)propane], 2,2'-azobis[2-(2-imidazolin-2-yl)propane]dihydrochloride and 2,2'-azobis[2-(2-imidazolin-2-yl)propane]disulfate dihydrate.

Preferred examples of the water-soluble peroxide is any of potassium persulfate, ammonium persulfate, sodium persulfate, tertiary butyl hydroperoxide, cumyl hydroperoxide, paramentane hydroperoxide and diisopropylbenzene hydroperoxide.

In the present embodiment, the monomer represented by formula (X1) is preferable for sensitivity improvement because the refractive index of the polymer obtained by polymerizing the monomer is high. Examples of the monomer include styrenes, 1-vinylnaphthalene and 2-vinylnaphthalene, and in particular, styrene, 1-vinylnaphthalene and 2-vinylnaphthalene are preferred. These monomers may be used singly, or a plurality of these monomers may be used at the same time.

Styrenes: styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, p-phenylstyrene and the like.

In the present embodiment, the monomer represented by formula (X2) has a glycidyl group in the side chain, so that the glycidyl group in the shell containing the polymer of formula (X2) can react with the compound represented by formula (X3), and a carboxy group or a hydroxy contained in formula (X3) can be introduced onto the surface of the particle. The monomer represented by formula (X2) is not particularly limited, but glycidyl (meth)acrylate is preferred.

In the present embodiment, the monomer represented by formula (X3) is attached to the shell on the surface of the particle by reacting in a third step an amino group or a thiol group in formula (X3) with a glycidyl group in the shell to form formula (2). The monomer represented by formula (X3) is not particularly limited, and examples include mercaptosuccinic acid, aspartic acid, 3-mercapto-1,2-propanediol, 3-amino-1,2-propanediol, 2-amino-1,3-propanediol, ethanolamine and tris-hydroxymethylamino methane.

### Third Embodiment

The present disclosure, as a third embodiment, provides the following particle.

A particle for use in an agglutination method, produced by:
a first step of performing a polymerization reaction of a first monomer composition containing 40% by mass or more and 85% by mass or less of a monomer represented by formula (X1) to obtain a core particle having a volume average particle size of 200 nm or more and 500 nm or less;
a second step of performing a polymerization reaction using a reaction solution comprising the core particle, a second monomer composition comprising a monomer represented by formula (X2) and a water-soluble polymerization initiator to obtain a particle having a layer of 5 nm or more and 50 nm or less formed outside of the core particle,
a content portion of the monomer represented by the formula (X1) to a total amount of the monomer contained in the first monomer composition is 90 mass% or more
provided that,
a content proportion of an unpolymerized monomer represented by the formula (X1) to the reaction solution in the second step is 1000 ppm or less, and
a content proportion of the monomer represented by the formula (X2) to a total amount of a monomer contained in the first monomer composition and a monomer contained in the second monomer composition is 9% by mass or more and less than 40% by mass; and
a third step of reacting the particle having the layer with a compound represented by formula (X3) 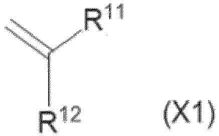
   (wherein, R¹¹ represents a hydrogen atom or a methyl group; and R¹² represents a substituted or unsubstituted phenyl or naphthyl group, provided that, when substituted, a substituent is a methyl group or an ethyl group) 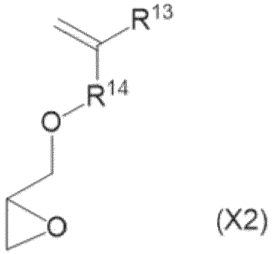
   (wherein, R¹³ represents hydrogen or a methyl group, and R¹⁴ represents an ethylene group or a carbonyl group) 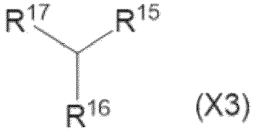
   (wherein, R¹⁵ represents an amino group or a thiol group; R¹⁶ and R¹⁷ each represent a hydrogen atom, a methyl group, a group having a hydroxy group or a group having a carboxy group; and at least one of R¹⁶ and R¹⁷ represents a group having a hydroxy group or a group having a carboxy group).

### Other Embodiments

The present disclosure provides, as a further embodiment, a test particle including a ligand attached to a surface of the particle of the present disclosure as described above.

A test particle in the present disclosure has a selective or specific, high affinity for a target substance by means of a ligand attached to the surface of the particle. In particular, it is preferred that the test particle is one in which the ligand is attached to the surface of the particle by chemical bonding.

The ligand in the present disclosure is a compound that specifically binds to a receptor possessed by a certain target substance. The site at which the ligand binds to the target substance is fixed, and the ligand has a selective or specific, high affinity. Examples include an antigen and an antibody, an enzyme protein and a substrate thereof, a signaling substance such as hormone or neurotransmitter and a receptor thereof and a nucleic acid, but the ligand in the present disclosure is not limited to these. Examples of the nucleic acid include a deoxyribonucleic acid. The test particle in the present disclosure has a selective or specific, high affinity for a target substance. It is preferable that the ligand in the present disclosure is any of an antibody, an antigen and a nucleic acid.

The present disclosure provides, as a further embodiment, a reagent for use in an agglutination method characterized in that the particle or test particle of the present disclosure as described above is dispersed in an aqueous solution. The test object of the reagent is not particularly limited. Examples of the reagent include a medicament for in vitro diagnostics, and specifically include an antigen detection reagent for testing an antigen and an antibody detection reagent for testing an antibody. The reagent may be one in which a particle without a ligand is dispersed in an aqueous solution, assuming that a user attaches a ligand such as a desired antibody to the particle. Alternatively, the reagent may be one in which a test particle to which a ligand is attached in advance is dispersed in an aqueous solution, assuming a specific target substance.

The reagent of the present disclosure includes the particle or test particle of the present disclosure and a dispersion medium for dispersing the particle or the test particle. The reagent of the present disclosure may contain a third substance such as a solvent, a blocking agent or the like in addition to the particle or test particle of the present disclosure, to the extent that the object of the present disclosure can be achieved. The third substance such as a solvent or a blocking agent may be contained in combination with two or more types. Examples of the dispersion medium used in the present disclosure include various buffers such as a phosphate buffer, a glycine buffer, a Good buffer, a Tris buffer and an ammonia buffer, but the dispersion medium contained in the reagent of the present disclosure is not limited to these.

The present disclosure provides, as a further embodiment, a test kit. The kit includes the reagent described above and a container enclosing the reagent.

The kit in the present embodiment may include a reaction buffer (hereinafter, reagent 2) in addition to the reagent described above (hereinafter, reagent 1). Both or one of the reagent 1 and the reagent 2 may contain a sensitizing agent. The kit of the present disclosure may also include a positive control, a negative control, a serum dilution, a primary antibody, a secondary antibody or the like in addition to the reagent 1 and the reagent 2. As a medium for the positive control and the negative control, serum that does not contain a target substance that may be measured, saline or a solvent may be used.

The present disclosure provides, as a further embodiment, a method for detecting a target substance in a sample by in vitro diagnostics, the method including mixing the reagent containing the test particle described above with a sample that may contain the target substance.

The present disclosure provides, as a further embodiment, a method for detecting a target substance in a sample by an agglutination method. The agglutination method according to the present embodiment is a method for detecting a target substance in a sample, including a step of mixing the reagent containing the test particle described above with a sample to obtain a mixed solution; a step of irradiating the mixed solution with light; and a step of detecting at least one of transmitted light or scattered light from the light with which the mixed solution has been irradiated.

### [Measurement method of volume average particle size and particle size distribution of particle in aqueous dispersion]

A method for measuring a volume average particle size (Dv) in an aqueous dispersion of a particle in the present disclosure will be described. The Dv of particles present in the aqueous dispersion is measured by dynamic light scattering method. For example, the measurement is performed using a zetasizer (Zetasizer Ultra: Malvern Panalytical Ltd.) at 25°C. The Dv of the core particles is measured with an aqueous dispersion of the core particles obtained in the first step described above. The Dv of the particles having a core and shell structure is measured with an aqueous dispersion of the particles obtained in the third step described above.

The particle size distribution of the particles having the core and shell structure of the present disclosure is obtained by measuring the number average particle size (Dn) by the dynamic light scattering method described above, and calculating the ratio of Dv to Dn (Dv/Dn).

### [Measurement method of thickness of shell of particle]

A method for calculating a thickness of a shell of a particle in the present disclosure will be described. The thickness of the shell is calculated by subtracting the volume average particle size of the core particle from the volume average particle size of the particle obtained by measuring the water dispersion of the particle obtained in the third step described above, and dividing by 2.

### [Measurement method of composition ratio (C/O) of C element to O element of particle]

The measurement of the composition ratio (C/O) of C element to O element in the present disclosure will be described. The composition ratio of the particle of the present disclosure by X-ray photoelectron spectroscopy (XPS) is measured using a lyophilized particle fixed to an indium foil. In Examples described below, the following measurement apparatus and measurement conditions were used.
- Measurement apparatus: X-ray photoelectron spectrometer: Quantum 2000 (product name, manufactured by ULVAC-PHI, Inc.)
- X-ray source: monochrome AlKα
- Xray setting: 100 µmϕ (25 W (15 KV))
- Photoelectron take-off angle: 45 degrees
- Neutralization condition: combination of a neutralizing gun and an ionizing gun
- Analysis area: 300 × 200 µm
- Pass energy: 58.70 eV
- Step size: 0.125 eV
- Analysis software: Maltipak (ULVAC-PHI, Inc.)

The cumulative number at the time of measurement is 15 times for C1S and O1S, and 30 times for N1S. The obtained quantitative values of the amount of elements (the ratio (atomic %) of the amount of C element and the ratio (atomic %) of the amount of O element each to the total amount of the three elements) is used to determine the composition ratio of C element to O element.

### [Measurement method of infrared absorption spectrum of particle]

A method for measuring a ratio of C=O bond from an ester to C=C bond from an aromatic ring in IR spectrum of the particle according to the present disclosure, that is, a ratio of an ester component to an aromatic ring component in the particle, will be described. The component ratio of the particle according to the present disclosure by IR spectrum is measured using a lyophilized particle. In Examples described below, the following measurement apparatus and measurement conditions were used.
- Measurement apparatus: Fourier transform infrared spectrometer Spectrum One (PerkinElmer, Inc.)
- Measurement method: ATR method
- Crystal: Diamond
- Measurement range: 4000 cm⁻¹ to 650 cm⁻¹
- Resolution: 4 cm⁻¹
- Cumulative number: 32 times
- Data analysis: performed with absorbance

Lyophilized, powdery particles are placed on the measurement section, and a flat probe is used to press them down. At that time, the measurement is started so that the pressure gauge displayed on the measurement software is about 100.

After the measurement, quantification and analysis are performed with absorbance data.

The highest peak height present at 1500 to 1650 cm⁻¹ derived from the C=C bond of the aromatic ring is defined as A and the highest peak height present at 1680 to 1750 cm⁻¹ derived from the carbonyl group of the ester bond is defined as B, and a value of A/B is calculated.

### [Measurement method of absorbance of particle]

A method for measuring the absorbance of a particle in the present disclosure will be described. The absorbance of the particle can be obtained by measuring the absorbance of the aqueous dispersion of the particle of the present disclosure at a wavelength of 572 nm. In the following Examples, a 0.01% by mass aqueous dispersion of the particle was added to a cell, and the absorbance at a cell length of 10 mm with incident light of 572 nm was measured using Biospectrometer, a spectrophotometer manufactured by Eppendorf corporate.

### Examples

Hereinafter, the present disclosure will be described in detail by way of Examples.

### [Example 1] (Synthesis of particle 1)

### (Step-1/Preparation of core 1)

Into a 2 L four-necked separable flask, 71.75 g of styrene (St: KISHIDA CHEMICAL CO., LTD.), 1.30 g of divinylbenzene (DVB: KISHIDA CHEMICAL CO., LTD.) and 1190.67 g of ion-exchanged water were weighed to prepare a mixed solution. This mixed solution was then held at 70°C while stirring at 140 rpm, and a nitrogen flow was performed at a flow rate of 200 ml/min to deoxidize the inside of the four-necked separable flask. Next, a separately prepared solution obtained by dissolving 3.11 g of V-50 (FUJIFILM Wako Pure Chemical Corporation) in 50 g of ion-exchanged water was added to the above mixed solution to start soap-free emulsion polymerization. After reacting for 48 hours from the start of polymerization, a dispersion of core particle (hereinafter referred to simply as core) 1 containing a copolymer of St and DVB was obtained. A portion of the dispersion was collected and evaluated using dynamic light scattering of core 1 (Zetasizer Ultra: Malvern Panalytical Ltd.), and it was shown that the volume average particle size was 330 nm.

### (Step-2/Preparation of mother particle 1)

The dispersion was adjusted with ion-exchanged water so that 148.29 g of the dispersion of core 1 having a solid content concentration of 2.0% was prepared. A portion of this dispersion was collected and the content of St and DVB was evaluated using gas chromatography, and it was shown that the content of St and DVB was 75 ppm. Next, 1.52 g of glycidyl methacrylate (GMA: KISHIDA CHEMICAL CO., LTD.) was added, and the mixture was held at 70°C while stirring at 100 rpm, and a nitrogen flow was performed at a flow rate of 200 ml/min to deoxidize the four-necked separable flask. Then, a separately prepared solution obtained by dissolving 0.018 g of V-50 in 1 g of ion-exchanged water was added to the above mixed solution to start the formation of the shell. By continuing the stirring for 17 hours after the start of the reaction, a dispersion containing mother particle 1 having a core and shell structure was obtained. After slowly cooling the above dispersion to room temperature, a portion of the dispersion was collected, and the polymerization conversion rate was evaluated using gas chromatography and confirmed to be substantially 100%.

### (Step-3/Preparation of particle 1 having core and shell structure)

To an aqueous dispersion containing mother particle 1, a pre-prepared aqueous solution in which mercaptosuccinic acid (MSA, FUJIFILM Wako Pure Chemical Corporation) and 3-mercapto-1,2-propanediol (MPD: FUJIFILM Wako Pure Chemical Corporation) were dissolved (3-mercapto-1,2-propanediol and mercaptosuccinic acid were 6 : 4 (mole fraction), and the total mol number of MSA and MPD was equal to the mol number of glycidyl methacrylate described above) was added, and then triethylamine (KISHIDA CHEMICAL CO., LTD.) was added to adjust pH to 10. The mixture was then warmed to 70°C while stirring at 200 rpm, and held in this state for an additional 18 hours to obtain a dispersion of particle 1 having a core and shell structure. The operation of separating particle 1 from the dispersion by a centrifuge and re-dispersing particle 1 in ion-exchanged water was repeated 8 times to purify particle 1, and finally particle 1 was stored in an aqueous dispersion adjusted so that particle 1 was 1.0% by mass.

The results of evaluating the particle physical properties of particle 1 are shown in Table 1. The amount of formula (1) of the core 1, the content of formula (2) in particle 1, the content of formula (2) in the shell, and the mass ratio of core 1 to particle 1 are shown in Table 2.

Particle 1 was also subjected to IR measurements. The peak height present at 1500 to 1650 cm⁻¹ derived from the C=C bond of the aromatic ring was defined as A and the peak height present at 1680 to 1750 cm⁻¹ derived from the carbonyl of the ester bond was defined as B, and the value of A/B was calculated, which resulted in 4.05.

### [Table 1]

**Table 1**

| | | Particle | Volume average particle size Dv of core (nm) | thickness of shell (nm) | Particle size distribution Dv/Dn of particle (-) | Ratio E/Dv of absorbance (E) to Dv of particle (-) |
|---|---|---|---|---|---|---|
| Example | Example 1 | particle 1 | 330 | 20 | 1.15 | 1.4.E-03 |
| | Example 2 | particle 2 | 395 | 6 | 1.14 | 1.6.E-03 |
| | Example 3 | particle 3 | 498 | 40 | 1.16 | 1.7.E-03 |
| | Example 4 | particle 4 | 203 | 15 | 1.15 | 8.2.E-04 |
| | Example 5 | particle 5 | 332 | 15 | 1.14 | 1.4.E-03 |
| | Example 6 | particle 6 | 329 | 20 | 1.15 | 1.4.E-03 |
| | Example 7 | particle 7 | 331 | 20 | 1.16 | 1.4.E-03 |
| | Example 8 | particle 8 | 330 | 19 | 1.15 | 1.4.E-03 |
| | Example 9 | particle 9 | 330 | 20 | 1.13 | 1.4.E-03 |
| | Example 10 | particle 10 | 330 | 23 | 1.15 | 1.4.E-03 |
| | Example 11 | particle 11 | 333 | 22 | 1.14 | 1.4.E-03 |
| | Example 12 | particle 12 | 330 | 18 | 1.15 | 1.5.E-03 |
| | Example 13 | particle 13 | 330 | 16 | 1.15 | 1.5.E-03 |
| | Example 14 | particle 14 | 330 | 20 | 1.16 | 1.4.E-03 |
| | Example 15 | particle 15 | 450 | 20 | 1.16 | 1.9.E-03 |
| Comparative Example | Comparative Example 1 (deleted) | | | | | |
| | Comparative Example 2 | comparative particle 2 | 252 | - | 1.14 | 7.4.E-04 |
| | Comparative Example 3 | comparative particle 3 | 146 | 50 | 1.13 | 2.8.E-04 |
| | Comparative Example 4 | comparative particle 4 | 521 | 20 | 1.16 | 1.8.E-03 |
| | Comparative Example 5 | comparative particle 5 | 330 | 3 | 1.15 | 1.5.E-03 |
| | Comparative Example 6 | comparative particle 6 | 203 | 62 | 1.15 | 8.4.E-04 |

| | | | | | | |
|---|---|---|---|---|---|---|
| Ratio E/DV of absorbance (E) to Dv of particle: Value of E/DV when 0.1 wt% aqueous dispersion of particle is added to cell, and absorbance at cell length of 10 mm in incident light of 572 nm is set to E and volume average particle size of particle is set to DV. | | | | | | |

### [Table 2]

**Table 2**

| | | Particle | Monomer used during core particle production | Content of structure represented by formula (1) contained in core particle (% by weight) | Content of structure represented by formula (1) contained in particle (% by weight) | Content of structure represented by formula (2) contained in particle (% by weight) |
|---|---|---|---|---|---|---|
| Example | Example 1 | particle 1 | St, DVB | 98 | 53 | 45 |
| | Example 2 | particle 2 | St, DVB | 98 | 82 | 16 |
| | Example 3 | particle 3 | St, DVB | 98 | 45 | 53 |
| | Example 4 | particle 4 | St, DVB | 98 | 48 | 50 |
| | Example 5 | particle 5 | St, DVB | 93 | 61 | 37 |
| | Example 6 | particle 6 | St,IVN,DVB | 93 | 55 | 43 |
| | Example 7 | particle 7 | St,MSt,DVB | 98 | 53 | 45 |
| | Example 8 | particle 8 | St, DVB | 98 | 55 | 35 |
| | Example 9 | particle 9 | St, DVB | 98 | 54 | 37 |
| | Example 10 | particle 10 | St, DVB | 98 | 48 | 39 |
| | Example 11 | particle 11 | St, GMA | 98 | 51 | 47 |
| | Example 12 | particle 12 | St, DVB | 98 | 56 | 42 |
| | Example 13 | particle 13 | St, DVB | 98 | 59 | 42 |
| | Example 14 | particle 14 | St, DVB | 98 | 58 | 40 |
| | Example 15 | particle 15 | St, DVB | 98 | 61 | 37 |
| Comparative Example | Comparative Example 1 (deleted) | | | | | |
| | Comparative Example 2 | comparative particle 2 | St, DVB | 63 | 21 | - |
| | Comparative Example 3 | comparative particle 3 | St, DVB | 98 | 10 | 88 |
| | Comparative Example 4 | comparative particle 4 | St, DVB | 98 | 65 | 33 |
| | Comparative Example 5 | comparative particle 5 | St, DVB | 98 | 89 | 10 |
| | Comparative Example 6 | comparative particle 6 | St, DVB | 98 | 12 | 86 |

| | | Particle | Monomer used during core particle production | Content of structure represented by formula (2) contained in shell (% by weight) | Weight ratio of core to particle (% by weight) | Atomic mass ratio of C element to O element (mass ratio) |
|---|---|---|---|---|---|---|
| Example | Example 1 | particle 1 | St, DVB | 100 | 55 | 2.2 |
| | Example 2 | particle 2 | St, DVB | 100 | 84 | 2.1 |
| | Example 3 | particle 3 | St, DVB | 100 | 47 | 2.2 |
| | Example 4 | particle 4 | St, DVB | 100 | 50 | 2.2 |
| | Example 5 | particle 5 | St, DVB | 100 | 63 | 2.2 |
| | Example 6 | particle 6 | St,1VN,DVB | 100 | 57 | 2.2 |
| | Example 7 | particle 7 | St,MSt,DVB | 100 | 55 | 2.2 |
| | Example 8 | particle 8 | St, DVB | 100 | 57 | 2.2 |
| | Example 9 | particle 9 | St, DVB | 100 | 56 | 2.2 |
| | Example 10 | particle 10 | St, DVB | 100 | 50 | 2.2 |
| | Example 11 | particle 11 | St, GMA | 100 | 53 | 2.2 |
| | Example 12 | particle 12 | St, DVB | 93 | 58 | 23 |
| | Example 13 | particle 13 | St, DVB | 88 | 61 | 2.3 |
| | Example 14 | particle 14 | St, DVB | 100 | 60 | 2.2 |
| | Example 15 | particle 15 | St, DVB | 100 | 63 | 2.2 |
| Comparative Example | Comparative Example 1 (deleted) | | | | | |
| | Comparative Example 2 | comparative particle 2 | St, DVB | - | - | 3.4 |
| | Comparative Example 3 | comparative particle 3 | St, DVB | 100 | 12 | 2.2 |
| | Comparative Example 4 | comparative particle 4 | St, DVB | 100 | 67 | 2.2 |
| | Comparative Example 5 | comparative particle 5 | St, DVB | 100 | 90 | 3.5 |
| | Comparative Example 6 | comparative particle 6 | St, DVB | 100 | 14 | 2.2 |

### [Example 2] (Synthesis of particle 2)

### (Step-1/Preparation of core 2)

A dispersion of core 2 was obtained by the same experimental operation as in Example 1 except that the rotational speed of stirring in Example 1 was changed from 140 rpm to 200 rpm. A portion of the dispersion was collected and evaluated using dynamic light scattering of core 2 (Zetasizer Ultra: Malvern Panalytical Ltd.), and it was shown that the volume average particle size was 395 nm.

### (Step-2/Preparation of mother particle 2)

A dispersion of mother particle 2 was obtained by the same experimental operation except that the dispersion of core 1 was changed to a dispersion of core 2 in step-2 of Example 1. The content of St and DVB in the dispersion of core 2 before adding GMA was 83 ppm. The polymerization conversion rate was evaluated using gas chromatography and confirmed to be substantially 100%.

### (Step-3/Preparation of particle 3 having core and shell structure)

A dispersion of particle 2 having a core and shell structure was obtained by the same experimental operation as in step-3 of Example 1.

The results of evaluating the particle physical properties of particle 2 are shown in Table 1. The amount of formula (1) of the core 2, the content of formula (2) in particle 2, the content of formula (2) in the shell, and the mass ratio of core 2 to particle 2 are shown in Table 2.

### [Example 3] (Synthesis of particle 3)

### (Step-1/Preparation of core 3)

A dispersion of core 3 was obtained by the same experimental operation as in Example 1 except that the amount of St used was set to 99.40 g, the amount of DVB was set to 1.80 g, the amount of ion-exchanged water was set to 1150.19 g, and the amount of V-50 was set to 4.31 g in step-1 of Example 1, and the rotational speed of stirring was changed from 140 rpm to 200 rpm. A portion of the dispersion was collected and evaluated using dynamic light scattering of core 3 (Zetasizer Ultra: Malvern Panalytical Ltd.), and it was shown that the volume average particle size was 498 nm.

### (Step-2/Preparation of mother particle 3)

The dispersion was adjusted with ion-exchanged water so that 148.27 g of the dispersion of core 3 having a solid content concentration of 2.5% was prepared. A portion of this dispersion was collected and the content of St and DVB was evaluated using gas chromatography, and it was shown that the content of St and DVB was 82 ppm. Next, 2.37 g of GMA was added, and the mixture was held at 70°C while stirring at 100 rpm, and a nitrogen flow was performed at a flow rate of 200 ml/min to deoxidize the four-necked separable flask. Then, a separately prepared solution obtained by dissolving 0.018 g of V-50 (FUJIFILM Wako Pure Chemical Corporation) in 1 g of ion-exchanged water was added to the above mixed solution to start the formation of the shell. By continuing the stirring for 17 hours after the start of the reaction, a dispersion containing mother particle 3 having a core and shell structure was obtained. After slowly cooling the above dispersion to room temperature, a portion of the dispersion was collected, and the polymerization conversion rate was evaluated using gas chromatography and confirmed to be substantially 100%.

### (Step-3/Preparation of particle 3 having core and shell structure)

A dispersion of particle 3 having a core and shell structure was obtained by the same experimental operation as in step-3 of Example 1.

The results of evaluating the particle physical properties of particle 3 are shown in Table 1. The amount of formula (1) of the core 3, the content of formula (2) in particle 3, the content of formula (2) in the shell, and the mass ratio of core 3 to particle 3 are shown in Table 2.

### [Example 4] (Synthesis of particle 4)

### (Step-1/Preparation of core 4)

Into a 2 L four-necked separable flask, 25.35 g of styrene (St: KISHIDA CHEMICAL CO., LTD.), 0.46 g of divinylbenzene (DVB: KISHIDA CHEMICAL CO., LTD.) and 1501.02 g of ion-exchanged water were weighed to prepare a mixed solution. This mixed solution was then held at 70°C while stirring at 140 rpm, and a nitrogen flow was performed at a flow rate of 200 ml/min to deoxidize the inside of the four-necked separable flask. Next, a separately prepared solution obtained by dissolving 1.10 g of V-50 (FUJIFILM Wako Pure Chemical Corporation) in 30 g of ion-exchanged water was added to the above mixed solution to start soap-free emulsion polymerization. By reacting and stirring for 48 hours from the start of polymerization, a dispersion of core 4 containing a copolymer of St and DVB was obtained. A portion of the dispersion was collected and evaluated using dynamic light scattering of core 4 (Zetasizer Ultra: Malvern Panalytical Ltd.), and it was shown that the volume average particle size was 203 nm.

### (Step-2/Preparation of mother particle 4)

The dispersion was adjusted with ion-exchanged water so that 133.0 g of the dispersion of core 4 having a solid content concentration of 2.0% was prepared. A portion of this dispersion was collected and the content of St and DVB was evaluated using gas chromatography, and it was shown that the content of St and DVB was 20 ppm. Next, 1.43 g of GMA was added, and the mixture was held at 70°C while stirring at 100 rpm, and a nitrogen flow was performed at a flow rate of 200 ml/min to deoxidize the four-necked separable flask. Then, a separately prepared solution obtained by dissolving 0.0323 g of V-50 (FUJIFILM Wako Pure Chemical Corporation) in 1 g of ion-exchanged water was added to the above mixed solution to start the formation of the shell. By continuing the stirring for 17 hours after the start of the reaction, a dispersion containing mother particle 4 having a core and shell structure was obtained. After slowly cooling the above dispersion to room temperature, a portion of the dispersion was collected, and the polymerization conversion rate was evaluated using gas chromatography and confirmed to be substantially 100%.

### (Step-3/Preparation of particle 4 having core and shell structure)

A dispersion of particle 4 having a core and shell structure was obtained by the same experimental operation as in step-3 of Example 1.

The results of evaluating the particle physical properties of particle 4 are shown in Table 1. The amount of formula (1) of the core 4, the content of formula (2) in particle 4, the content of formula (2) in the shell, and the mass ratio of core 4 to particle 4 are shown in Table 2.

### [Example 5] (Synthesis of Particle 5)

### (Step-1/Preparation of core 5)

A dispersion of core 5 was obtained by the same experimental operation except that the amount of St used was changed from 71.75 g to 67.58 g and the amount of DVB used was changed from 1.30 g to 5.02 g in step-1 of Example 1. A portion of the dispersion was collected and evaluated using dynamic light scattering of core 5 (Zetasizer Ultra: Malvern Panalytical Ltd.), and it was shown that the volume average particle size was 332 nm.

### (Step-2/Preparation of mother particle 5)

A dispersion of mother particle 5 was obtained by the same experimental operation except that the amount of GMA used was changed from 1.52 g to 0.99 g and the amount of V-50 used was changed from 0.018 g to 0.016 g in step-2 of Example 1. The content of St and DVB in the dispersion of core 5 before adding GMA was 83 ppm. The polymerization conversion rate was evaluated using gas chromatography and confirmed to be substantially 100%.

### (Step-3/Preparation of particle 5 having core and shell structure)

A dispersion of particle 5 having a core and shell structure was obtained by the same experimental operation as in step-3 of Example 1.

The results of evaluating the particle physical properties of particle 5 are shown in Table 1. The amount of formula (1) of the core 5, the content of formula (2) in particle 5, the content of formula (2) in the shell, and the mass ratio of core 5 to particle 5 are shown in Table 2.

### [Example 6] (Synthesis of particle 6)

### (Step-1/Preparation of core 6)

A dispersion of core 6 was obtained by the same experimental operation except that 35.88 g of St and 35.88 g of 1-vinylnaphthalene (1VN: FUJIFILM Wako Pure Chemical Corporation) were used instead of 71.75 g of St amount in step -1 of Example 1. A portion of the dispersion was collected and evaluated using dynamic light scattering of core 6 (Zetasizer Ultra: Malvern Panalytical Ltd.), and it was shown that the volume average particle size was 329 nm.

### (Step-2/Preparation of mother particle 6)

A dispersion of mother particle 6 was obtained by the same experimental operation except that the dispersion of core 1 was changed to the dispersion of core 6 in step-2 of Example 1. The content of St and DVB in the dispersion of core 6 before adding GMA was 75 ppm. The polymerization conversion rate was evaluated using gas chromatography and confirmed to be substantially 100%.

### (Step-3/Preparation of particle 6 having core and shell structure)

A dispersion of particle 6 having a core and shell structure was obtained by the same experimental operation as in step-3 of Example 1.

The results of evaluating the particle physical properties of particle 6 are shown in Table 1. The amount of formula (1) of the core 6, the content of formula (2) in particle 6, the content of formula (2) in the shell, and the mass ratio of core 6 to particle 6 are shown in Table 2.

### [Example 7] (Synthesis of particle 7)

### (Step-1/Preparation of core 7)

A dispersion of core 7 was obtained by the same experimental operation except that 35.88 g of St and 35.88 g of 4-methylstyrene (MSt: FUJIFILM Wako Pure Chemical Corporation) were used instead of 71.75 g of St amount in step-1 of Example 1. A portion of the dispersion was collected and evaluated using dynamic light scattering of core 7 (Zetasizer Ultra: Malvern Panalytical Ltd.), and it was shown that the volume average particle size was 331 nm.

### (Step-2/Preparation of mother particle 7)

A dispersion of mother particle 7 was obtained by the same experimental operation except that the dispersion of core 1 was changed to the dispersion of core 7 in step-2 of Example 1. The content of St and DVB in the dispersion of core 7 before adding GMA was 88 ppm. The polymerization conversion rate was evaluated using gas chromatography and confirmed to be substantially 100%.

### (Step-3/Preparation of particle 7 having core and shell structure)

A dispersion of particle 7 having a core and shell structure was obtained by the same experimental operation as in step-3 of Example 1.

The results of evaluating the particle physical properties of particle 7 are shown in Table 1. The amount of formula (1) of the core 7, the content of formula (2) in particle 7, the content of formula (2) in the shell, and the mass ratio of core 7 to particle 7 are shown in Table 2.

### [Example 8] (Synthesis of particle 8)

A dispersion of particle 8 having a core and shell structure was obtained by the same experimental operation as in Example 1 except that mother particle 1 made in Example 1 was used and 3-amino-1,2-propanediol (3APD: Tokyo Chemical Industry Co., Ltd.) was used instead of MPD in step-3 of Example 1.

The results of evaluating the particle physical properties of particle 8 are shown in Table 1. The amount of formula (1) of the core 1, the content of formula (2) in particle 8, the content of formula (2) in the shell, and the mass ratio of core 1 to particle 8 are shown in Table 2.

### [Example 9] (Synthesis of particle 9)

A dispersion of particle 9 having a core and shell structure was obtained by the same experimental operation as in Example 1, except that mother particle 1 made in Example 1 was used and 2-amino-1,3-propanediol (2APD: Tokyo Chemical Industry Co., Ltd.) was used instead of MPD in step-3 of Example 1.

The results of evaluating the particle physical properties of particle 9 are shown in Table 1. The amount of formula (1) of the core 1, the content of formula (2) in particle 9, the content of formula (2) in the shell, and the mass ratio of core 1 to particle 9 are shown in Table 2.

### [Example 10] (Synthesis of particle 10)

A dispersion of particle 10 having a core and shell structure was obtained by the same experimental operation as in Example 1, except that mother particle 1 made in Example 1 was used and 2-amino-2-hydroxymethyl-1,3-propanediol (Tris: KISHIDA CHEMICAL CO., LTD.) was used instead of MPD in step-3 of Example 1.

The results of evaluating the particle physical properties of particle 10 are shown in Table 1. The amount of formula (1) of the core 1, the content of formula (2) in particle 10, the content of formula (2) in the shell, and the mass ratio of core 1 to particle 10 are shown in Table 2.

### [Example 11] (Synthesis of particle 11)

A dispersion of particle 11 having a core and shell structure was obtained by the same experimental operation as in Example 1, except that 1.42 g of GMA was used instead of DVB in step-3 of Example 1.

The results of evaluating the particle physical properties of particle 11 are shown in Table 1. The amount of formula (1) of the core 1, the content of formula (2) in particle 11, the content of formula (2) in the shell, and the mass ratio of core 1 to particle 11 are shown in Table 2.

### [Example 12] (Synthesis of Particle 12)

A dispersion of particle 12 having a core and shell structure was obtained by the same experimental operation as in Example 1, except that 0.67 g of GMA and 0.05 g of DVB were used instead of 0.72 g of GMA used in step-2 of Example 1.

The results of evaluating the particle physical properties of particle 12 are shown in Table 1. The amount of formula (1) of the core 12, the content of formula (2) in particle 12, the content of formula (2) in the shell, and the mass ratio of core 12 to particle 12 are shown in Table 2.

### [Example 13] (Synthesis of particle 13)

A dispersion of particle 13 having a core and shell structure was obtained by the same experimental operation as in Example 1, except that 0.63 g of GMA and 0.09 g of DVB were used instead of 0.72 g of GMA used in step-2 of Example 1.

The results of evaluating the particle physical properties of particle 13 are shown in Table 1. The amount of formula (1) of the core 13, the content of formula (2) in particle 13, the content of formula (2) in the shell, and the mass ratio of core 13 to particle 13 are shown in Table 2.

### [Example 14] (Synthesis of particle 14)

A dispersion of particle 14 having a core and shell structure was obtained by the same experimental operation as in Example 1, except that the total mol number of MSA and MPD used in step-3 of Example 1 was changed to 0.5 equivalents instead of the mol number and equivalent of GMA in step-2.

The results of evaluating the particle physical properties of particle 14 are shown in Table 1. The amount of formula (1) of the core 14, the content of formula (2) in particle 14, the content of formula (2) in the shell, and the mass ratio of core 14 to particle 14 are shown in Table 2.

### [Example 15] (Particle 15)

In step-1 of Example 1, the amount of St used was set to 85.61 g, the amount of DVB used was set to 1.55 g, the amount of ion-exchanged water used was 1120.12 g, and the amount of V-50 used was set to 3.72 g, and further the rotational speed of stirring was changed from 140 rpm to 200 rpm. In addition, in step-2 of Example 1, the amount of GMA used was changed to 0.45 g. Otherwise, a dispersion of core 15 was obtained by the same experimental operation as in Example 1. A portion of the dispersion was collected and evaluated using dynamic light scattering of core 15 (Zetasizer Ultra: Malvern Panalytical Ltd.), and it was shown that the volume average particle size was 498 nm.

### [Comparative Example 1] (deleted)

### [Comparative Example 2] (Synthesis of comparative particle 2)

St, GMA, DVB, V-50 and ion-exchanged water were subjected to polymerization reaction at 70°C for 24 hours after nitrogen substitution with the following formulation of St/GMA/DVB/V-50/H2O = 1.2/1.8 + 0.3/0.04/0.06/110 (g). The polymerization was the soap-free emulsion polymerization described in Japanese Patent Application Laid-Open No. 2000-351814. Two hours after the start of polymerization, 0.3 g of GMA was added, and the surface of the obtained comparative mother particle 2 was completely covered with GMA. The resulting comparative mother particle 2 was precipitated by centrifugation (15,000 rpm, 15 min, 4°C) and the supernatant was decanted and then redispersed in 200 ml of water. The above operation was repeated three times to wash comparative mother particle 2, and finally the resulting comparative mother particle 2 was dispersed in water.

To introduce an amino group into this washed comparative mother particle 2 (0.25 g), NH₄OH (55.3 mmol; equivalent to 50 times the amount of GMA unit) was added and adjusted to pH = 11 with 1N HCl. The mixture was reacted at 70°C for 24 hours while stirring with a stirrer to ring-open the epoxy group of GMA. Next, an example of immobilization of ethylene glycol diglycidyl ether (EGDE; FUJIFILM Wako Pure Chemical Corporation) in comparative mother particle 2 obtained above is shown. An excess EGDE was added to be in an amount (mol) of 100-fold the amount of the amino group of about 62.5 mg of comparative mother particle 2 and stirred at 30°C for 24 hours at pH 11 (adjusted with 1N NaOH) to covalently bond the epoxy group of EGDE with the amino group on comparative mother particle 2. An excess amount of EGDE was added to prevent the epoxy groups at both ends of one EGDE molecule from being immobilized to the SG particles at the same time. This resulted in EGDE particle in which EGDE was bound to mother particle 2. After reaction, the EGDE particle was washed three times with water by centrifugation.

To introduce an amino group into 0.25 g of this washed EGDE particle, NH₄OH (55.3 mmol; equivalent to 50 times the amount of GMA unit) was added and adjusted to pH = 1 with 1N HCl. The mixture was reacted at 70°C for 24 hours while stirring with a stirrer. Then, it was centrifuged with ion-exchanged water at 4°C under conditions of 3 times of 27000 G for 20 minutes, and then redispersed into methanol so that the solid content was 1% by mass. Next, 0.88 g of anhydrous succinic acid (Tokyo Chemical Industry Co., Ltd.) was added to the dispersion 2' weighed to contain 0.20 g of particles. The mixture was shaken at 30°C for 5 hours to react the primary amine of the particle in which the amino group was introduced at the EGDE end with anhydrous succinic acid to obtain comparative particle 2.

The results of evaluating the particle physical properties of comparative particle 2 are shown in Table 1.

### [Comparative Example 3] (Synthesis of comparative particle 3)

Comparative core 3 was obtained by the same experimental operation as in step-1 of Example 4, except that 12.68 g of St, 0.23 g of DVB and 1501.02 g of ion-exchanged water were weighed into a 2 L four-necked separable flask in step-1 of Example 1 to prepare a mixed solution. Then, comparative particle 3 was obtained by the same experimental operation as in step 2 and step 3 of Example 4 except that 1.31 g of GMA was used instead of 1.43 g of GMA in step-2 of Example 4.

The results of evaluating the particle physical properties of comparative particle 3 are shown in Table 1.

### [Comparative Example 4] (Synthesis of comparative particle 4)

A dispersion of comparative core 4 was obtained by the same experimental operation as in Example 1 except that 99.40 g of St, 1.80 g of DVB and 1150.15 g of ion-exchanged water were weighed into a 2 L four-necked separable flask to prepare a mixed solution, the rotational speed of stirring was changed from 140 rpm to 200 rpm, and the addition amount of V-50 was changed to 4.31 g in step-1 of Example 1. Then, comparative particle 4 was obtained by the same experimental operation as in step-2 and step-3 of Example 1.

The results of evaluating the particle physical properties of comparative particle 4 are shown in Table 1.

### [Comparative Example 5] (Synthesis of comparative particle 5)

Comparative particle 5 was obtained by the same experimental operation as in Example 1, except that 0.092 g of GMA was used instead of 1.52 g of GMA in step-2 of Example 1.

The results of evaluating the particle physical properties of comparative particle 5 are shown in Table 1.

### [Comparative Example 6] (Synthesis of Comparative Particle 6)

Comparative particle 6 was obtained by the same experimental operation as in Example 1, except that 10.01 g of GMA was used instead of 1.43 g of GMA in step-2 of Example 4.

The results of evaluating the particle physical properties of comparative particle 6 are shown in Table 1.

For Comparative Examples 1 to 6, the amount of formula (1) of the core, the content of formula (2) in the comparative particle, the content of formula (2) in the shell, and the mass ratio of the core to the comparative particle are shown in Table 2.

### [Evaluation 1] Preparation of test particle by antibody sensitization to particle and evaluation of agglutination sensitivity of test particle

### (Preparation of test particle by antibody sensitization to particle)

For each of particles 1 to 15 and comparative particles 1 to 6 prepared in Examples 1 to 15 and Comparative Examples 1 to 6, 180 µL of 1.7% by mass water-suspension was taken into a 1.5 mL microtube, then 90 µL of a 5.0% aqueous solution of 1-(3-dimethylaminopropyl)-3-ethylcarbodiimide hydrochloride and 90 µL of a 5.0% aqueous solution of N-hydroxysulfosuccinimide sodium hydrochloride were added thereto, and the mixture was stirred at room temperature for 30 minutes to activate the carboxy group, thereby obtaining a particle dispersion (activated particle dispersion).

After centrifugal washing, 270 µL of phosphate buffer-saline solution (hereinafter PBS) at pH 7.2 was added, and the above particles were dispersed with ultrasonic wave.

5 µL of 15.0 mg/mL dispersion of clone C5 of monoclonal mouse anti-human C-reactive protein (anti-CRP antibody) (Funakoshi Co., Ltd.) was added thereto, and the mixture was stirred at room temperature for 3 hours to sensitize the antibody to the particles, thereby obtaining a test particle. Each of these test particles was centrifugally washed, then 1 mL of PBS was added, and the test particles were stored in a dispersed state.

### (Evaluation of agglutination sensitivity of test particle)

The standard serum for CRP was diluted with PBS to be the concentration of 0.75 mg/dL, which was taken as CRP sample solution. One µL of the CRP sample solution was mixed with 50 µL of buffer (PBS containing 0.01% Tween 20) to prepare a mixed solution (hereinafter referred to as R1+), which was incubated at 37°C.

Next, R1+ was mixed with 50 µL of dispersion prepared by fully dispersing each of the test particle dispersion with ultrasonic wave again before use (particle concentration: 0.1% by mass, referred to as R2). The absorbance at a wavelength of 572 nm was measured for the mixed solution (volume: 101 µL) immediately after stirring. The absorbance measurement was performed using Biospectrometer, a spectrophotometer manufactured by Eppendorf corporate. The mixed solution was then allowed to stand at 37°C for 5 minutes, and then the absorbance at the wavelength of 572 nm was measured again, and the value of absorbance change amount ΔABS × 10000 (R+) was calculated. The results are summarized in Table 3.

It is expected that the higher the value of R+ of the test particle, the more sensitively the target substance can be detected when the test particle is used in sample testing as a particle for agglutination method.

Table 3 shows the evaluation results of test particles of particles 1 to 15 and comparative particles 1 to 6. For any of particles 1 to 15, the value of absorbance change amount ΔABS × 10000 was 10000 or more.

On the other hand, for any of comparative particles 1 to 3, the value of ΔABS × 10000 was less than 10000. For comparative particles 1 and 2, as shown in Table 2, the content of the structure represented by formula (1) in the core particle was 50% by mass and 63% by mass, respectively, thus the proportion of substances with low refractive index was higher than that of particles 1 to 15, so that the refractive index of the core particles was low.

As a result, the absorbance difference before and after agglutination became small, and the sensitivity was low. For comparative particle 3, as shown in Table 1, the particle size of the core was 146 nm in volume average particle size, which was smaller than that of the particles 1 to 15, thus the agglutinate of the particles after agglutination was small, so that the difference in absorbance before and after agglutination was small. In addition, the values of E/Dv of comparative particles 1 to 3 were less than 8.0 × 10⁻⁴, and even if the particle size was a certain size or larger, the absorbance was small, and the difference in the absorbance before and after agglutination was small. For comparative particle 4, the particle size of the core particle was 521 nm, which was larger than that of particles 1 to particle 15, and the value of ΔABS × 10000 was 7,000, thus the sensitivity was low. For comparative particle 6, the value of ΔABS × 10000 was low as 2000. This is because the thickness of the shell was thick as 62 nm, and the dispersibility of the particles was high, so it was difficult to cause agglutination.

### [Evaluation 2] Evaluation of non-specific adsorption for particle

### (Emulsion evaluation)

For particles 1 to 15 and comparative particles 1 to 6, a dispersion was prepared by dispersing each in a phosphate buffer to be 0.1% by mass. Next, for 30 µL of each dispersion, 60 µL of emulsion solution containing triolein, lecithin, free fatty acid, bovine albumin and tris buffer was added, and the absorbance at a wavelength of 572 nm was measured for the resulting dispersion immediately after stirring. The absorbance measurement was performed using Biospectrometer, a spectrophotometer manufactured by Eppendorf corporate. After these dispersions were allowed to stand at 37°C for 5 minutes, the absorbance at the wavelength of 572 nm was measured again, and the absorbance change amount ΔABS × 10000 was calculated. According to the value of ΔABS × 10000, they were evaluated as follows.
A: ΔABS × 10000 is 30 or less
B: ΔABS × 10000 is more than 30 and 50 or less
C: ΔABS × 10000 is more than 50 and 100 or less
D: ΔABS × 10000 is more than 100 and 500 or less
E: ΔABS × 10000 is more than 500

Table 3 shows the evaluation results of particles 1 to 15 and comparative particles 1 to 6. The evaluation results of particles 1 to 15 were all B or more. Since the change in absorbance of the dispersion can be considered to be due to the occurrence of interparticle agglutination as a result of non-specific adsorption of the particles in the dispersion, it was confirmed that the particles of the present disclosure are superior in their ability to suppress non-specific adsorption. On the other hand, the evaluation result of comparative particle 5 was E, thus non-specific adsorption could not be sufficiently suppressed. It is believed that this is because the thickness of the shell of comparative particle 5 was 3 nm and thinner than that of particles 1 to 15, so that a portion of the core particles that were hydrophobic was exposed to the surface of the particle in comparative particle 5.

### (Human sample evaluation)

For particles 1 to 15 and comparative particles 1 to 6, a dispersion (P solution) was prepared by dispersing each particle in a phosphate buffer (containing 0.01% Tween 20) to be 0.1% by mass. Next, for 50 µL of each dispersion, 55 µL of sample dilution (Q solution) containing a human normal sample (serum sample, 5 µL) and a phosphate buffer (50 µL) was added, and the absorbance at a wavelength of 572 nm was measured for the resulting mixed solution immediately after stirring. The absorbance measurement was performed using Biospectrometer, a spectrophotometer manufactured by Eppendorf corporate. After these dispersions were allowed to stand at 37°C for 5 minutes, the absorbance at the wavelength of 572 nm was measured again, and the absorbance change amount ΔABS × 10000 was calculated. According to the value of ΔABS × 10000, they were evaluated as follows.
A: ΔABS × 10000 is 30 or less
B: ΔABS × 10000 is more than 30 and 80 or less
C: ΔABS × 10000 is more than 80 and 100 or less
D: ΔABS × 10000 is more than 100 and 500 or less
E: ΔABS × 10000 is more than 500

The results of each particle are shown in Table 3.

Table 3 shows the evaluation results of particles 1 to 15 and comparative particles 1 to 6. The evaluation results of particles 1 to 15 were all A to C, and the value of the change amount ΔABS × 10000 was less than 100. Since the change in absorbance of the dispersion can be considered to be due to the occurrence of interparticle agglutination as a result of non-specific adsorption of the particles in the dispersion, it was confirmed that the particles of the present disclosure are superior in their ability to suppress non-specific adsorption. On the other hand, the evaluation result of comparative particle 5 was E and the value of change amount ΔABS × 10000 was 1000, thus non-specific adsorption could not be sufficiently suppressed. It is believed that this is because the thickness of the shell of comparative particle 5 was 3 nm and thinner than that of particles 1 to 15, and thus, a portion of the core particles that were hydrophobic was exposed to the surface of the particle in comparative particle 5.

As described above, the particles 1 to 15 of the present disclosure had high agglutination sensitivity from the results of Evaluation 1, and suppressed non-specific adsorption from the results of Evaluation 2, showing that both improvement of sensitivity and suppression of non-specific adsorption can be achieved. On the other hand, in Comparative Examples 1 to 4, non-specific adsorption was suppressed, but the agglutination sensitivity was low. Comparative Example 6 was less sensitive. In Comparative Example 5, although the agglutination sensitivity was high, non-specific adsorption could not be suppressed. In other words, the improvement of agglutination sensitivity and the suppression of non-specific adsorption could not be balanced in Comparative Examples 1 to 6.

### [Table 3]

**Table 3**

| | | Particle | [Evaluation 1] Evaluation of sensitivity in agglutination method | [Evaluation 2] Evaluation of non-specific adsorption for particle | |
|---|---|---|---|---|---|
| | | | ΔABS×10000 | (Emulsion evaluation) | (Human sample evaluation) |
| Example | Example 1 | particle 1 | 12000 | B | B |
| | Example 2 | particle 2 | 13000 | B | B |
| | Example 3 | particle 3 | 15000 | B | B |
| | Example 4 | particle 4 | 10000 | B | B |
| | Example 5 | particle 5 | 11800 | A | A |
| | Example 6 | particle 6 | 12500 | B | B |
| | Example 7 | particle 7 | 12000 | B | B |
| | Example 8 | particle 8 | 12000 | A | A |
| | Example 9 | particle 9 | 12000 | A | A |
| | Example 10 | particle 10 | 12000 | A | A |
| | Example 11 | particle 11 | 10000 | B | B |
| | Example 12 | particle 12 | 12000 | B | B |
| | Example 13 | particle 13 | 12000 | C | C |
| | Example 14 | particle 14 | 10000 | C | C |
| | Example 15 | particle 15 | 15000 | A | A |
| Comparative Example | Comparative Example 1 (deleted) | | | | |
| | Comparative Example 2 | comparative particle 2 | 8000 | B | B |
| | Comparative Example 3 | comparative particle 3 | 1500 | B | B |
| | Comparative Example 4 | comparative particle 4 | 7000 | B | B |
| | Comparative Example 5 | comparative particle 5 | 13000 | E | E |
| | Comparative Example 6 | comparative particle 6 | 2000 | D | D |

According to the present disclosure, by having a core and shell structure, having a high refractive index of the core particles, and having a reactive functional group in the shell, a particle having improved sensitivity and an ability to suppress non-specific adsorption in an agglutination method can be provided. Furthermore, a test particle, a reagent, a test kit, a method for detecting a target substance, and a method for producing the particle can be provided.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A particle for use in an agglutination method, the particle comprising a core particle and a shell on a surface of the core particle, wherein
the core particle has a volume average particle size of 200 nm or more and 500 nm or less, as measurable by dynamic light scattering, and contains a polymer having a structural unit represented by formula (1), wherein a content proportion of the structural unit represented by the formula (1) to the particle is 40% by mass or more and 85% by mass or less; and
the shell has a thickness of 5 nm or more and 50 nm or less, and contains a polymer having a structural unit represented by formula (2) 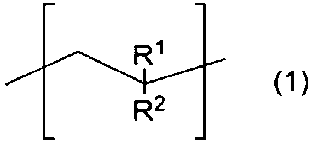
wherein, R¹ represents a hydrogen atom or a methyl group;
R² represents a substituted or unsubstituted phenyl or naphthyl group, provided that, when substituted, a substituent is a methyl group or an ethyl group; and
R¹ and R² may be different for each structural unit, 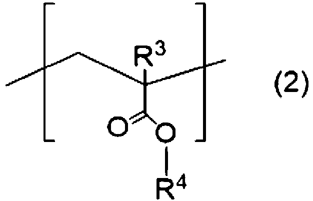
wherein, R³ represents a hydrogen atom or a methyl group;
R⁴ represents a group having an epoxy group, a group having a hydroxy group, or a group having a carboxy group; and
R³ and R⁴ may be different for each structural unit.

2. The particle according to claim 1, wherein a content of the structural unit represented by the formula (2) to the particle is 9% by mass or more and 60% by mass or less.

3. The particle according to claim 2, wherein a content of the structural unit represented by the formula (2) to the particle is 9% by mass or more and 39% by mass or less.

4. The particle according to claim 3, wherein a content of the structural unit represented by the formula (2) to the particle is 20% by mass or more and 39% by mass or less.

5. The particle according to any one of claims 1 to 4, wherein a content proportion of the structural unit represented by the formula (1) to the core particle is 90% by mass or more.

6. The particle according to any one of claims 1 to 5, wherein a composition ratio of C element to O element of the particle quantified when measured by X-ray photoelectron spectroscopy (XPS) is 2.0 or more and 3.3 or less.

7. The particle according to any one of claims 1 to 6, wherein a content proportion of the structural unit represented by the formula (2) to the shell is 90% by mass or more and 100% by mass or less.

8. The particle according to any one of claims 1 to 7, wherein the polymer having a structural unit represented by the formula (1) in the core particle has a structural unit represented by formula (3) 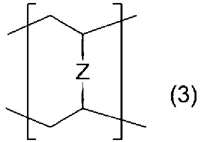 wherein, Z represents a substituted or unsubstituted phenylene or naphthalene group, provided that, when substituted, a substituent is a methyl group or an ethyl group; and Z may be different for each structural unit.

9. The particle according to any one of claims 1 to 8, wherein a content proportion of the core particle to the particle is 50% by mass or more and 95% by mass or less.

10. The particle according to any one of claims 1 to 9, wherein, when a peak height present at 1500 to 1650 cm⁻¹ derived from a C=C bond of an aromatic ring is defined as A and a peak height present at 1680 to 1750 cm⁻¹ derived from a carbonyl group of an ester bond is defined as B in an FT-IR spectrum of the particle, a value of A/B is 0.85 or more and 5.35 or less.

11. The particle according to any one of claims 1 to 10, wherein a value of a ratio (Dv/Dn) of a volume average particle size (Dv) to a number average particle size (Dn) of the particle is 1.25 or less.

12. The particle according to any one of claims 1 to 11, wherein, when the particle is dispersed in water so that a ratio (Dv/Dn) of a volume average particle size (Dv) to a number average particle size (Dn) is 1.25 or less at a solid content concentration of 0.1% by mass, and an absorbance at a cell length of 10 mm in an incident light of a wavelength of 572 nm is defined as E, a value of E/Dv is 8.0 × 10⁻⁴ or more and 2.0 × 10⁻³ or less.

13. The particle according to any one of claims 1 to 12, wherein the formula (1) is represented by formula (1-A) 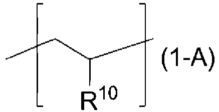 wherein, R¹⁰ represents a phenyl group, a tolyl group or a naphthyl group.

14. The particle according to any one of claims 1 to 13, wherein the formula (2) is represented by formula (2-A) 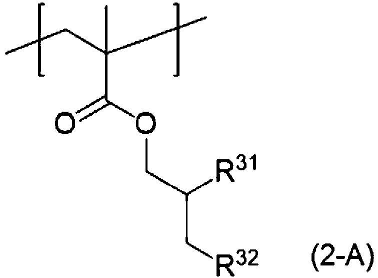
wherein, one of R³¹ and R³² represents a hydroxy group, and the other represents a hydroxy group, a group represented by formula (2-B) or a group represented by formula (2-C), 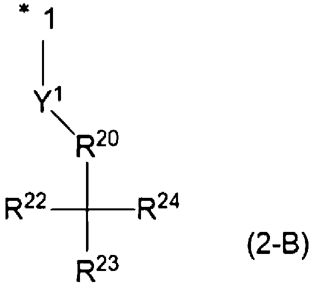
wherein, R²⁰ represents a single bond or a methylene group;
R²², R²³ and R²⁴ each represent a hydrogen atom, a methyl group, a hydroxy group or a hydroxymethyl group, and one or more of R²², R²³ and R²⁴ represent a hydroxy group;
Y¹ represents a sulfur atom or an imino group; and
*1 represents a bonding position, 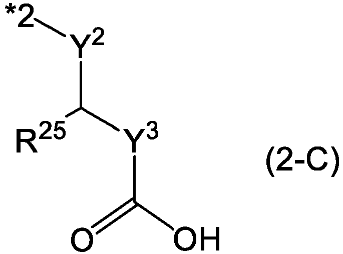
wherein, R²⁵ represents a hydrogen atom, a methyl group, a hydroxy group or a carboxy group;
Y² represents a sulfur atom or an imino group;
Y³ represents a single bond or a methylene group; and
*2 represents a bonding position.

15. A test particle comprising a ligand attached to a surface of the particle according to any one of claims 1 to 14.

16. The test particle according to claim 15 wherein the ligand is an antibody specific for C-reactive protein.

17. A reagent for use in an agglutination method, wherein the particle according to any one of claims 1 to 14 is dispersed in an aqueous solution.

18. A test kit comprising: the reagent according to claim 17; and a container enclosing the reagent.

## Patentansprüche

1. Partikel zur Verwendung in einem Agglutinationsverfahren, wobei das Partikel ein Kernpartikel und eine Hülle auf einer Oberfläche des Kernpartikels umfasst, wobei
das Kernpartikel eine volumengemittelte Partikelgröße von 200 nm oder mehr und 500 nm oder weniger, messbar durch dynamische Lichtstreuung, aufweist und ein Polymer mit einer durch Formel (1) dargestellten Struktureinheit enthält, wobei ein Gehaltsanteil der durch Formel (1) dargestellten Struktureinheit an dem Partikel 40 Massen-% oder mehr und 85 Massen-% oder weniger beträgt; und
die Hülle eine Dicke von 5 nm oder mehr und 50 nm oder weniger aufweist und ein Polymer mit einer durch Formel (2) dargestellten Struktureinheit enthält 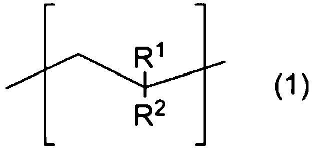
wobei R¹ ein Wasserstoffatom oder eine Methylgruppe darstellt;
R² eine substituierte oder unsubstituierte Phenyl- oder Naphthylgruppe darstellt, vorausgesetzt, dass, wenn substituiert, ein Substituent eine Methylgruppe oder eine Ethylgruppe ist; und
R¹ und R² für eine jeweilige Struktureinheit unterschiedlich sein können, 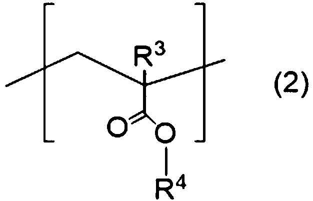
wobei R³ ein Wasserstoffatom oder eine Methylgruppe darstellt;
R⁴ eine Gruppe mit einer Epoxygruppe, eine Gruppe mit einer Hydroxygruppe oder eine Gruppe mit einer Carboxygruppe darstellt; und
R³ und R⁴ für eine jeweilige Struktureinheit unterschiedlich sein können.

2. Partikel nach Anspruch 1, wobei ein Gehalt der durch Formel (2) dargestellten Struktureinheit an dem Partikel 9 Massen-% oder mehr und 60 Massen-% oder weniger beträgt.

3. Partikel nach Anspruch 2, wobei ein Gehalt der durch Formel (2) dargestellten Struktureinheit zu dem Partikel 9 Massen-% oder mehr und 39 Massen-% oder weniger beträgt.

4. Partikel nach Anspruch 3, wobei ein Gehalt der durch Formel (2) dargestellten Struktureinheit an dem Partikel 20 Massen-% oder mehr und 39 Massen-% oder weniger beträgt.

5. Partikel nach einem der Ansprüche 1 bis 4, wobei ein Gehaltsanteil der durch Formel (1) dargestellten Struktureinheit an dem Kernpartikel 90 Massen-% oder mehr beträgt.

6. Partikel nach einem der Ansprüche 1 bis 5, wobei ein durch Messung durch Röntgenphotoelektronenspektroskopie (XPS) quantifiziertes Zusammensetzungsverhältnis von C-Element zu O-Element des Partikels, 2,0 oder mehr und 3,3 oder weniger beträgt.

7. Partikel nach einem der Ansprüche 1 bis 6, wobei ein Gehaltsanteil der durch Formel (2) dargestellten Struktureinheit an der Hülle 90 Massen-% oder mehr und 100 Massen-% oder weniger beträgt.

8. Partikel nach einem der Ansprüche 1 bis 7, wobei das Polymer mit einer durch Formel (1) dargestellten Struktureinheit in dem Kernpartikel eine durch Formel (3) dargestellte Struktureinheit aufweist 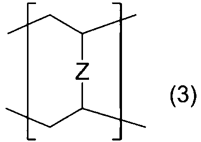 wobei Z eine substituierte oder unsubstituierte Phenylen- oder Naphthalingruppe darstellt, vorausgesetzt, dass, wenn substituiert, ein Substituent eine Methylgruppe oder eine Ethylgruppe ist; und wobei Z für eine jeweilige Struktureinheit unterschiedlich sein kann.

9. Partikel nach einem der Ansprüche 1 bis 8, wobei ein Gehaltsanteil des Kernpartikels an dem Partikel 50 Massen-% oder mehr und 95 Massen-% oder weniger beträgt.

10. Partikel nach einem der Ansprüche 1 bis 9, wobei, wenn in einem FT-IR-Spektrum des Partikels eine Peakhöhe, die bei 1500 bis 1650 cm⁻¹ vorliegt und von einer C=C-Bindung eines aromatischen Rings abgeleitet ist, als A definiert ist und eine Peakhöhe, die bei 1680 bis 1750 cm⁻¹ vorliegt und von einer Carbonylgruppe einer Esterbindung abgeleitet ist, als B definiert ist, ein Wert von A/B 0,85 oder mehr und 5,35 oder weniger beträgt.

11. Partikel nach einem der Ansprüche 1 bis 10, wobei ein Wert eines Verhältnisses (Dv/Dn) einer volumengemittelten Partikelgröße (Dv) zu einer zahlengemittelten Partikelgröße (Dn) des Partikels 1,25 oder weniger beträgt.

12. Partikel nach einem der Ansprüche 1 bis 11, wobei, wenn das Partikel in Wasser dispergiert ist, so dass ein Verhältnis (Dv/Dn) einer volumengemittelten Partikelgröße (Dv) zu einer zahlengemittelten Partikelgröße (Dn) bei einer Feststoffgehaltkonzentration von 0,1 Massen-% 1,25 oder weniger beträgt, und wenn bei einer Küvettenlänge von 10 mm eine Extinktion einfallenden Lichts einer Wellenlänge von 572 nm als E definiert ist, ein Wert von E/Dv 8,0 x 10⁻⁴ oder mehr und 2,0 x 10⁻³ oder weniger beträgt.

13. Partikel nach einem der Ansprüche 1 bis 12, wobei die Formel (1) durch Formel (1-A) dargestellt ist 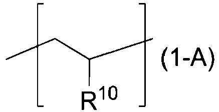 wobei R¹⁰ eine Phenylgruppe, eine Tolylgruppe oder eine Naphthylgruppe darstellt.

14. Partikel nach einem der Ansprüche 1 bis 13, wobei die Formel (2) durch Formel (2-A) dargestellt ist 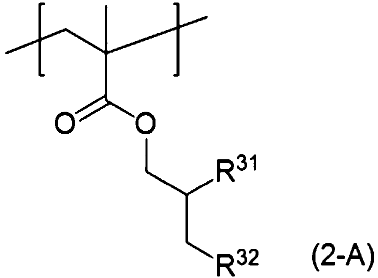
wobei eines von R³¹ und R³² eine Hydroxygruppe darstellt und das andere eine Hydroxygruppe, eine durch Formel (2-B) dargestellte Gruppe oder eine durch Formel (2-C) dargestellte Gruppe darstellt 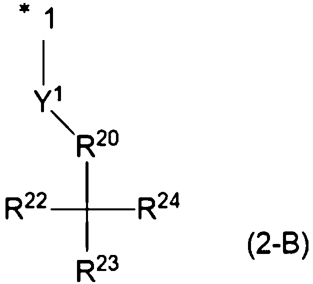
wobei R²⁰ eine Einfachbindung oder eine Methylengruppe darstellt;
R²², R²³ und R²⁴ jeweils ein Wasserstoffatom, eine Methylgruppe, eine Hydroxygruppe oder eine Hydroxymethylgruppe darstellen und eines oder mehrere von R²², R²³ und R²⁴ eine Hydroxygruppe darstellen;
Y¹ ein Schwefelatom oder eine Iminogruppe darstellt; und
*1 eine Bindungsposition darstellt 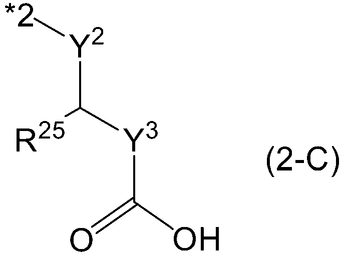
wobei R²⁵ ein Wasserstoffatom, eine Methylgruppe, eine Hydroxygruppe oder eine Carboxygruppe darstellt;
Y² ein Schwefelatom oder eine Iminogruppe darstellt;
Y³ eine Einfachbindung oder eine Methylengruppe darstellt; und
*2 eine Bindungsposition darstellt.

15. Testpartikel, umfassend einen Liganden, der an eine Oberfläche des Partikels nach einem der Ansprüche 1 bis 14 gebunden ist.

16. Testpartikel nach Anspruch 15, wobei der Ligand ein für C-reaktives Protein spezifischer Antikörper ist.

17. Reagens zur Verwendung in einem Agglutinationsverfahren, wobei das Partikel nach einem der Ansprüche 1 bis 14 in einer wässrigen Lösung dispergiert ist.

18. Testkit, umfassend: das Reagens nach Anspruch 17; und einen Behälter, der das Reagens beinhaltet.

## Revendications

1. Particule pour une utilisation dans un procédé d'agglutination, la particule comprenant une particule de cœur et une écorce sur une surface de la particule de cœur, dans laquelle
la particule de cœur possède une granulométrie moyenne en volume de 200 nm ou plus et de 500 nm ou moins, telle que mesurable par diffusion dynamique de la lumière, et contient un polymère ayant une unité structurale représentée par la formule (1), dans laquelle une proportion de teneur de l'unité structurale représentée par la formule (1) sur la particule est de 40 % en masse ou plus et de 85 % en masse ou moins ; et
l'écorce possède une épaisseur de 5 nm ou plus et de 50 nm ou moins, et contient un polymère ayant une unité structurale représentée par la formule (2) 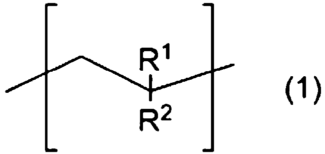
dans laquelle, R¹ représente un atome d'hydrogène ou un groupe méthyle ;
R² représente un groupe phényle ou naphtyle substitué ou non substitué, à condition que, lorsqu'il est substitué, un substituant soit un groupe méthyle ou un groupe éthyle ; et
R¹ et R² peuvent être différents pour chaque unité structurale, 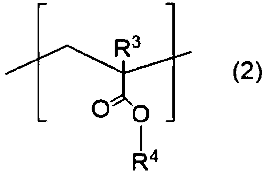
dans laquelle, R³ représente un atome d'hydrogène ou un groupe méthyle ;
R⁴ représente un groupe ayant un groupe époxy, un groupe ayant un groupe hydroxy,
ou un groupe ayant un groupe carboxy ; et
R³ et R⁴ peuvent être différents pour chaque unité structurale.

2. Particule selon la revendication 1, dans laquelle une teneur de l'unité structurale représentée par la formule (2) sur la particule est de 9 % en masse ou plus et de 60 % en masse ou moins.

3. Particule selon la revendication 2, dans laquelle une teneur de l'unité structurale représentée par la formule (2) sur la particule est de 9 % en masse ou plus et de 39 % en masse ou moins.

4. Particule selon la revendication 3, dans laquelle une teneur de l'unité structurale représentée par la formule (2) sur la particule est de 20 % en masse ou plus et de 39 % en masse ou moins.

5. Particule selon l'une quelconque des revendications 1 à 4, dans laquelle une proportion de teneur de l'unité structurale représentée par la formule (1) sur la particule de cœur est de 90 % en masse ou plus.

6. Particule selon l'une quelconque des revendications 1 à 5, dans laquelle un rapport de composition de l'élément C sur l'élément O de la particule, quantifié lorsqu'il est mesuré par spectroscopie photoélectronique X (XPS), est de 2,0 ou plus et de 3,3 ou moins.

7. Particule selon l'une quelconque des revendications 1 à 6, dans laquelle une proportion de teneur de l'unité structurale représentée par la formule (2) sur l'écorce est de 90 % en masse ou plus et de 100 % en masse ou moins.

8. Particule selon l'une quelconque des revendications 1 à 7, dans laquelle le polymère ayant une unité structurale représentée par la formule (1) dans la particule de cœur possède une unité structurale représentée par la formule (3) 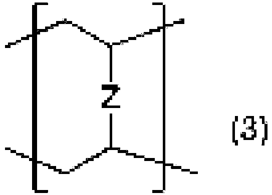 dans laquelle, Z représente un groupe phénylène ou naphtalène substitué ou non substitué, à condition que, lorsqu'il est substitué, un substituant soit un groupe méthyle ou un groupe éthyle ; et Z peut être différent pour chaque unité structurale.

9. Particule selon l'une quelconque des revendications 1 à 8, dans laquelle une proportion de teneur de la particule de cœur sur la particule est de 50 % en masse ou plus et de 95 % en masse ou moins.

10. Particule selon l'une quelconque des revendications 1 à 9, dans laquelle, lorsqu'une hauteur de pic présente de 1500 à 1650 cm⁻¹ issue d'une liaison C=C d'un cycle aromatique est définie comme étant A et une hauteur de pic présente de 1680 à 1750 cm⁻¹ issue d'un groupe carbonyle d'une liaison ester est définie comme étant B dans un spectre infrarouge à transformée de Fourier, FT-IR, de la particule, une valeur de A/B est de 0,85 ou plus et de 5,35 ou moins.

11. Particule selon l'une quelconque des revendications 1 à 10, dans laquelle une valeur d'un rapport (Dv/Dn) d'une granulométrie moyenne en volume (Dv) sur une granulométrie moyenne en nombre (Dn) de la particule est de 1,25 ou moins.

12. Particule selon l'une quelconque des revendications 1 à 11, dans laquelle, lorsque la particule est dispersée dans l'eau, de sorte qu'un rapport (Dv/Dn) d'une granulométrie moyenne en volume (Dv) sur une granulométrie moyenne en nombre (Dn) soit de 1,25 ou moins à une concentration de teneur en solides de 0,1 % en masse, et qu'une absorbance avec une longueur de cuve de 10 mm sous une lumière incidente d'une longueur d'onde de 572 nm soit définie comme étant E, une valeur de E/Dv est de 8,0 x 10⁻⁴ ou plus et de 2,0 x 10⁻³ ou moins.

13. Particule selon l'une quelconque des revendications 1 à 12, dans laquelle la formule (1) est représentée par la formule (1-A) 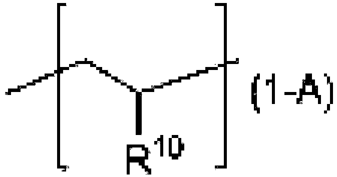 dans laquelle, R¹⁰ représente un groupe phényle, un groupe tolyle ou un groupe naphtyle.

14. Particule selon l'une quelconque des revendications 1 à 13, dans laquelle la formule (2) est représentée par la formule (2-A) 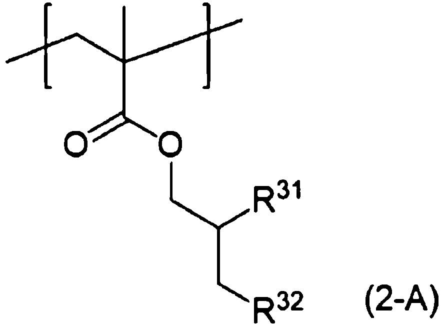
dans laquelle, l'un parmi R³¹ et R³² représente un groupe hydroxy, et l'autre représente un groupe hydroxy, un groupe représenté par la formule (2-B) ou un groupe représenté par la formule (2-C), 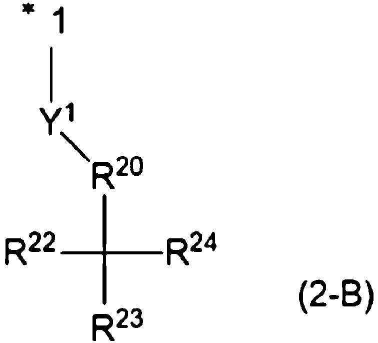
dans laquelle, R²⁰ représente une liaison simple ou un groupe méthylène ;
R²², R²³ et R²⁴ représentent chacun un atome d'hydrogène, un groupe méthyle, un groupe hydroxy ou un groupe hydroxyméthyle, et l'un ou plusieurs parmi R²², R²³ et R²⁴ représentent un groupe hydroxy ;
Y¹ représente un atome de soufre ou un groupe imino ; et
*1 représente une position de liaison, 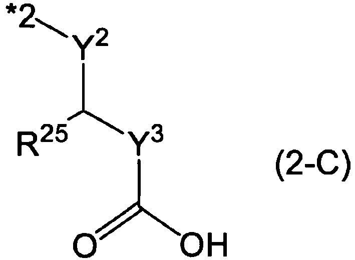
dans laquelle, R²⁵ représente un atome d'hydrogène, un groupe méthyle, un groupe hydroxy ou un groupe carboxy ; Y² représente un atome de soufre ou un groupe imino ;
Y³ représente une liaison simple ou un groupe méthylène ; et
*2 représente une position de liaison.

15. Particule de test comprenant un ligand fixé à une surface de la particule selon l'une quelconque des revendications 1 à 14.

16. Particule de test selon la revendication 15 dans laquelle le ligand est un anticorps spécifique à la protéine C réactive.

17. Réactif pour une utilisation dans un procédé d'agglutination, dans lequel la particule selon l'une quelconque des revendications 1 à 14 est dispersée dans une solution aqueuse.

18. Kit de test comprenant : le réactif selon la revendication 17 ; et un contenant renfermant le réactif.
